# EUROPEAN PATENT SPECIFICATION

(11) **EP 3 301 189 B1**
(45) Date of publication and mention of the grant of the patent: **04.11.2020**
(21) Application number: 17193458.1
(22) Date of filing: 27.09.2017
(51) Int. Cl.: C12Q 1/689

(54) **MOLECULAR METHOD FOR THE AUTHENTICATION OF TRADITIONAL/TYPICAL BREADS OBTAINED WITH SOURDOUGH, PRIMERS USED IN SAID METHOD AND KIT CONTAINING SAID PRIMERS**
MOLEKULARES VERFAHREN ZUR AUTHENTIFIZIERUNG VON TRADITIONELLEM / TYPISCHEM SAUERTEIGBROT, DAZU VERWENDETE PRIMER UND KIT ENTHALTEND DIESE PRIMER
PROCÉDÉ MOLÉCULAIRE POUR L'AUTHENTIFICATION DE PAINS TRADITIONNELS / TYPIQUES AU LEVAIN, AMORCES UTILISÉES DANS LEDIT PROCÉDÉ ET KIT CONTENANT CES AMORCES

(30) Priority: 29.09.2016 IT 201600097963
(43) Date of publication of application: 04.04.2018
(73) Proprietor: Università degli Studi di Bari "Aldo Moro", 70121 Bari (BA) (IT)
(72) Inventor: GOBBETTI, Marco, I-06134 Perugia PG (IT); PONTONIO, Erica, I-71016 San Severo FG (IT); DI CAGNO, Raffaella, I-70126 Bari BA (IT); LANERA, Alessia, I-70019 Triggiano BA (IT); DE ANGELIS, Maria, I-64012 Campli TE (IT); VAN SINDEREN, Douwe, Carrigrohane, Country Cork (IE)
(74) Representative: Trupiano, Federica

(56) References cited:
- WO-A1-2013/003262
- MELANIE WIESCHEBROCK ET AL: "Quantitative detection of lactic acid bacteria in dried sourdoughs using real-time PCR", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 233, no. 4, 12 August 2011 (2011-08-12), pages 617-624, XP019955414, ISSN: 1438-2385, DOI: 10.1007/S00217-011-1537-2
- F. MINERVINI ET AL: "Lactic Acid Bacterium and Yeast Microbiotas of 19 Sourdoughs Used for Traditional/Typical Italian Breads: Interactions between Ingredients and Microbial Species Diversity", APPLIED AND ENVIRONMENTAL MICROBIOLOGY, vol. 78, no. 4, 15 February 2012 (2012-02-15), pages 1251-1264, XP055178050, ISSN: 0099-2240, DOI: 10.1128/AEM.07721-11
- NICOLAS GRYSON ET AL: "PCR detection of soy ingredients in bread", EUROPEAN FOOD RESEARCH AND TECHNOLOGY ; ZEITSCHRIFT FÜR LEBENSMITTELUNTERSUCHUNG UND -FORSCHUNG A, SPRINGER, BERLIN, DE, vol. 227, no. 2, 1 August 2007 (2007-08-01), pages 345-351, XP019621688, ISSN: 1438-2385

### SEQUENCE LISTING

<110> Università degli Studi di Bari Aldo Moro
<120> Molecular method for the authentication of traditional/typical breads obtained with sourdough, primers used in said method and kit containing said primers
<130> 170912D35
<160> 2
<170> PatentIn version 3.5
<210> 1
   <211> 21
   <212> DNA
   <213> Artificial Sequence
<220>
   <223> Primer SKfw
<400> 1
   ggggataaca yytggaaaca g 21
<210> 2
   <211> 23
   <212> DNA
   <213> Artificial Sequence
<220>
   <223> Primer SKrw
<400> 2
   ctcggctacg tatcattgtc ttg 23

## Description

### Abstract of the Invention

Object of the present invention is a method for the authentication of traditional/typical breads, i.e. breads leavened with sourdough (mother yeast, mother paste or acid paste), by a molecular method allowing the detection and quantification of the DNA of lactic acid bacteria possibly present in the bread. In particular, the invention relates to the use of primers allowing to carry out a quantitative polymerase chain reaction (qPCR) allowing the cellular density of bacteria, advantageously lactic acid bacteria, contained in the leavened food products to be verified, and to determine if such a density is compatible with natural leavening. The above mentioned primers and a kit containing thereof are also an object of the invention.

### Technical Field

Although handicraft and, especially, industrial bakeries also use other leavening agents, the potential of the sourdough (also named mother yeast, mother paste, acid paste) have been widely demonstrated. The sourdough, the use of which is widespread at national and international level, can be considered as one of the most ancient examples of traditional biotechnology applied to food transformations. The natural leavening positively affects the stiffness, taste, flavor, preservability, and the nutritional and functional characteristics of the bakery products.

The sourdough is defined as a mixture of flour (mainly from cereals) and water, fermented by a metabolically active and heterogeneous population of lactic acid bacteria and native yeasts. The fermentation is usually spontaneous or rarely driven by the addition of selected starters. As it is known, the preparation of the sourdough needs different fermentation steps, usually characterized by incubation temperatures between 30 and 37°C, and subsequent backslopping. After the initial fermentation, a part (5 - 30%) of the dough previously fermented is added to additional flour and water, so to maintain the microorganisms metabolically active. The flour or the other ingredients used for the preparation of the sourdough are not microbiologically sterile nor they are thermally treated prior to use. For such a reason, when the flour is kneaded with water and the dough is subjected to incubation, contaminant microorganisms grow and the cellular density of the more competitive species reaches definitely high values. At the beginning of the first fermentation, the microbial population of the dough reflects that one present in the flour and mainly consists of lactic acid bacteria, sporogenous bacteria of genus *Bacillus, Pseudomonas* sp., *Enterobacteriaceae,* yeasts and molds.

Each of these microbial groups is present at cellular density not higher than 4-5 log cfu/g. As a consequence of the fermentation operated by lactic acid bacteria, the pH value of the dough at the end of the process is definitely acid. Usually, this decrease is very evident after the first backslopping and causes the inhibition of the *Enterobacteriaceae* population. The yeasts, on the other hand, are able to tolerate such acidic conditions. Consequently, with the increase of the number of backsloppings, environmental conditions selectively favoring the dominance of lactic acid bacteria and yeasts are created.

A mature sourdough contains cellular densities of lactic acid bacteria and yeasts, respectively, of about 7 - 9 log cfu/g and about 5 - 8 log cfu/g. The main metabolic activities made by this microbial consortium are the acidification (lactic acid bacteria), the formation of volatile compounds responsible for the taste (yeasts and, especially, lactic acid bacteria), and the leavening/volume increase of the dough (lactic acid bacteria and, especially, yeasts). An abundant literature demonstrated that structural, taste, flavor, preservability, nutritional and functional characteristics of bakery products obtained by natural leavening are higher than those of other products obtained by other types of leavening, and are almost uniquely linked to the metabolism of lactic acid bacteria.

The evolution, microbial composition and stability of the microbiota of a sourdough are influenced by a number of factors. A number of studies highlighted that the ingredients, the environmental contamination wherein the yeast is prepared and propagated, and the process parameters (e.g. amount of water, fermentation time and temperature) have fundamental role on stability and composition of the microbiota of the sourdough. Although the microbial diversity (number of species and biotypes) of a sourdough may considerably vary, the richness in lactic acid bacteria, expressed in terms of cellular density, is never less than about 7.0 log cfu/g. Independently from the above mentioned factors, the lactic acid bacteria species that usually dominates the sourdoughs belong to the *Enterococcus*, *Lactococcus*, *Leuconostoc*, *Pediococcus*, *Weissella* and, especially, *Lactobacillus* genera.

European producers and consumers are continuously rediscovering traditional and typical food products of their own Countries, greatly appreciating the nutritional, organoleptic, qualitative and cultural aspects. The enormous diversification of the European agri-food heritage has been recognized by the European Community, leading to the creation of different types of mark of origin for agricultural products and derivatives (EU Regulation 2081/1992). Within the category of the agricultural products and derivatives, cereals derivatives play an important role as they are perfectly integrated in the daily diet. On average, the yearly European consumption of bread is equal to about 58 kg per capita, and varies from 120 kg in Turkey to 32 kg in the UK (AIBI Bread Market Report, 2015). There are a number of European leavened bakery products (about 30 - 50%) that, by tradition, provide the use of the sourdough and as such need protection. All the breads enjoying the status of Protected Designation of Origin ("Denominazione di Origine Protetta") (Altamura Bread "Pane di Altamura" and Loaf of the Dittaino "Pagnotta del Dittaino") or Protected Geographical Indication ("Indicazione Geografica Protetta") (Matera Bread "Pane di Matera", Coppia Ferrarese and Genzano House-made Bread "Pane casareccio di Genzano") are obtained by the natural leavening. In Italy, there are about 200 types of different traditional/typical breads, all of which are produced by natural leavening.

Another category of products, for which the natural leavening represents the main link with tradition/typicality, is that one of the confectionery products, both for the recurrences (such as for example Panettone, Pandoro, Pizza pasquale, Panaredda and Colomba) and consumed daily. The annual production is estimated to be equal to about 114,250 t (ISTAT, 2015).

Food fraud is one of the main causes of economic loss of the industrial sector and consumer distrust. The use of sourdough requires the work of highly specialized personnel and rather long processing times. This influences the price of the products that, as they are depicted on the labels, seem to be more expensive than the counterpart of the products obtained by brewer's yeast or chemical leavening agent. In general, consumers appreciating such productions are willing to recognize a higher market price. Consequently, declaring the presence of sourdough in a product not containing it, not only is a fraud but also allows the producers to place the product at a higher level, thus obtaining an increase in earnings without an actual increase of production costs.

The perception of the consumer and the legislative protection of the term "sourdough" in Germany, France and other European countries (Austria, Denmark, Sweden and Norway) (FEDIMA, 2014) have eased the recent revival and the growing use of the natural leavening. Although the sourdough is one of the few intermediate products of the food production that is regulated by legislation and recognized by many consumers, after the cooking step there isn't any method allowing the sourdough authentication in the bread. To date, the authentication of bread with natural leavening is based on the manufacturer's label statement. The only confirmation is of indirect nature, by the determination of the pH value or concentration of lactic acid and acetic acid or in terms of rheological and sensory quality subjectively perceived by consumers. None of the European or other country legislations rules and/or authenticates the use of sourdough in bread-making. The only exception is represented by the French legislation that defines the production of *"pain au Levain",* and establishes the pH value and concentration of organic acids of the product (FEDIMA 2014; Décret n°93-1074 del 1993). However, also in this case nothing is established about the cellular density of lactic acid bacteria that has to be reached at the end of the fermentation and indirectly determined in the cooked products. Despite the diversity of the sourdoughs, almost twenty years ago a consensus has been reached about the number of vital lactic acid bacteria that, following the natural leavening, must be reached at the end of the fermentation. It has to be at least equal to 7.0 log cfu/g.

Melanie Wieschebrock et al. disclose a real-time PCR system to quantitatively detect lactic acid bacteria of the genera *Lactobacillus*, *Leuconostoc*, *Pediococcus*, and *Weissella* in different types of commercially available dried sourdoughs. Said real-time PCR was designed to amplify a 341bp 16S rRNA gene fragment of the sourdough lactic acid bacteria biota.

WO2013003262 describes a real time PCR system to identify and/or quantify lactic acid bacteria in food products as yogurt, vegetables, deli style meat products, and dairy prod fish products. Said real time PCR is based on the amplification of 16S rRNA through a primer pair and a probe both complementary to regions of 16S rRNA gene Minervini et al. have studied the microbiotas of 19 italian sourdoughs used for the manufacture of traditional/typical breads through a culture-dependent approach, to identify 20 species of lactic acid bacteria (LAB) and 4 of yeasts.

Based on what has been described, the need to have access to quick and effective methods to ascertain the presence of lactic acid bacteria in the declared natural leavened traditional/typical breads is therefore evident and necessary, thus allowing the authentication of the technology and product and the protection of the consumers who appreciate such productions and are willing to recognize a higher market price.

### Objects of the Invention

It is an object of the invention to provide a method for the detection and quantitative determination of lactic acid bacteria contained in leavened food products.

It is another object of the invention to provide a method for differentiating food products leavened with mother yeast from food products leavened with brewer's yeast or chemical yeast.

It is another object of the invention to provide a primer pair and the use thereof in the method of the invention.

It is further object of the invention to provide a kit comprising the above mentioned primers.

### BRIEF DESCRIPTION OF THE FIGURES

Figure 1 depicts the agarose gel of amplified genomic DNA extracted from lactic acid bacteria and contaminants of the flour by using the method of the invention.
Figure 2 depicts the standard curve generated with the C_{T} values of breads containing serial dilutions of a pure culture of *Lactobacillus plantarum* and the logarithm of the copies of the 16S rRNA gene (log gene copies/g) of the same microorganism.
Figure 3 depicts the Scatterplot based on the values of the Nr. (wherein Nr. = number) of copies of the 16S rRNA gene (log gene copies/g) of breads produced at the pilot plant of the Dipartimento di Scienze del Suolo, della Pianta e degli Alimenti (DiSSPA).
Figure 4 depicts the Scatterplot based on the values of the number of copies of the 16S rRNA gene (log gene copies/g) of breads purchased at the bakeries and supermarkets.
Figures 5 A and B depict the Principal Component Analysis (PCA) based on the values of number of copies of the 16S rRNA gene (log gene copies/g), pH, TTA (Total Titratable Acidity) and lactic and acetic acid concentrations of lab (A) and commercial (B) breads with natural leavening or produced by using brewer's yeast only.

### Description of the Invention

Object of the present invention is a method for the detection of lactic acid bacteria in leavened food products, comprising:
a. extracting the total DNA from a sample of said leavened food product;
b. carrying out a quantitative polymerase chain reaction (qPCR) on the DNA extracted in step (a) by using primers specific for bacterial 16S rRNA sequences, said primer pair being SKfw (5'-GGGGATAACAYYTGGAAACAG-3') (SEQ. ID NO. 1) and SKrw (5'-CTCGGCTACGTATCATTGTCTTG-3') (SEQ. ID NO. 2).
c. determining the CT value;
d. determining, by means of the equation of the calibration line, the value of the number of copies of the 16S rRNA gene expressed as log gene copies/g, wherein the value of the number of copies of the 16S rRNA > 7 log gene copies/g identifies a food product leavened with sourdough.

As it is known, the CT value (threshold cycle, commonly denoted as "threshold" or also "cut-off" in English) is inversely proportional to the concentration at the amount of target gene present in the assayed sample.

According to the invention, the method for the detection and quantitative determination of bacteria, in particular lactic acid bacteria, comprises carrying out the steps (a) to (d) mentioned above, wherein a value of the number of copies of the 16S rRNA gene higher than 7 log gene copies/g identifies a significant presence of bacteria, in particular lactic acid bacteria, and thus a food product leavened with sourdough.

The above described method allows food products leavened with mother yeast to be differentiated from food products leavened with brewer's yeast or chemical yeast.

The method of the invention can therefore be used as a method for differentiating food products leavened with sourdough from food products leavened with brewer's yeast or chemical yeasts, and comprises carrying out the above mentioned steps (a) to (d), wherein a value of number of copies of the 16S rRNA gene higher than 7 log gene copies/g identifies a food product compatible with a food product leavened with sourdough.

It is also an object of the invention a method for differentiating food products leavened with sourdough from food products leavened with brewer's yeast or chemical yeasts, comprising carrying out the steps (a) to (d) mentioned above, wherein a value of number of copies of the 16S rRNA gene lower than 7 log gene copies/g identifies a food product leavened with brewer's yeast or chemical yeasts.

In the method of the invention the determination of the value is carried out by setting the "threshold" to a value between 0.1 and 1, preferably between 0.3 and 0.5, advantageously 0.4.

According to the invention, in the methods of the invention the primers are specific for the amplification of 16S rRNA sequences of lactic acid bacteria.

In the method of the invention step (b) is carried out by using the oligonucleotide pair SKfw (5'-GGGGATAACAYYTGGAAACAG-3') (SEQ. ID NO. 1) and SKrw (5'-CTCGGCTACGTATCATTGTCTTG-3') (SEQ. ID NO. 2) as a primer which amplifies a region of about 178 bp of the 16S rRNA of the lactic acid bacteria which are usually in the food products leavened with sourdough, advantageously lactic acid bacteria belonging to the *Lactobacillus*, *Lactococcus*, *Leusonostoc*, *Pediococcus* and *Weissella* genera.

The oligonucleotides mentioned above constitute a further object of the invention as well as the use thereof as qPCR primers for the detection and quantitative determination of lactic acid bacteria, in particular but not only, in the method of the invention.

Object of the invention are said oligonucleotides as primers for the detection and quantitative determination of lactic acid bacteria, in leavened food products, advantageously in the bread.

By "leavened food product" is meant herewith to denote the bread, sandwich loaf or any other bakery product, such as leavened sweet or salty pies, leavened sweets such as cookies, panettone, pandoro, colomba, etc. Preferably the leavened food product is bread.

The extraction of the DNA of step (a) can be carried out by techniques known to the skilled in the art, by using kits available in the market. Examples of such kits are mentioned in the Experimental Section of the present description.

In the same way, qPCR can be carried out by techniques known to the skilled in the art, by using kits available in the market.

According to a preferred embodiment, the amplification process of the methods of the invention comprises:
- a reaction mixture (25 µl) constituted by: (i) 12.5 µl of Rotor-Gene SYBR Green RT-PCR master mix, (ii) 0.75 µl of each primer (SKfw/SKrw) at a concentration of 100 µM, (iii) 10 µl of RNase free water and (iv) 1 µl of DNA;
- an amplification cycle carried out in the Rotor-Gene 6000 (Corbett Research Ltd., Australia) which includes a first denaturation step of the DNA at 95°C for 10 minutes followed by 35 cycles of: (i) denaturation at 95°C for 10 seconds; (ii) annealing at 55°C for 30 seconds; and (iii) elongation at 72°C for 30 seconds and, to complete the amplification, a "melting curve" in a temperature range of 60 - 95°C, with 1°C increments;
- a quantification carried out by using the Rotor-Gene 6000 Series Software, version 1.7, by manually setting the "threshold" at a value of 0.4. Anyway, other amplification processes can be used.

According to another of its aspects, object of the invention is a kit for the detection and quantitative determination of lactic acid bacteria in leavened food products by qPCR comprising the primer pair
SKfw (5'-GGGGATAACAYYTGGAAACAG-3') (SEQ. ID NO. 1) and
SKrw (5'-CTCGGCTACGTATCATTGTCTTG-3') (SEQ. ID NO. 2).

As demonstrated in the following Experimental Section, the method of the invention allows the authentication of traditional/typical breads, i.e. breads leavened with sourdough, in a quick and precise way, therefore solving the technical problem and allowing to show up food frauds.

### Experimental section

### Methods

According to the present invention, an amount variable between 0.1 and 10 g (preferably 1 g) of fresh or frozen bread (preferably fresh) or other leavened bakery product is subjected to extraction of the total DNA by using a commercial kit for isolating and purifying the DNA from food matrices, preferably the Wizard Magnetic DNA Purification System for Food (Promega, Madison WI). In detail, after weighing an amount of 1 g bread in a test tube with the volume of 50 ml it is necessary: (i) adding 2.5 ml lysis solution (A) and 25 µl RNase, and vigorously stirring; (ii) adding 1.25 ml lysis solution (B), vigorously stirring and incubating at room temperature for 10-15 minutes; (iii) adding 3.75 ml precipitation solution, vigorously stirring and centrifuging at 10,000 rpm for 20 minutes; (iv) collecting the supernatant and adding 100 µl magnetic beads, after taking note of the volume of supernatant; (v) adding 0.8% (v/v) isopropanol and vigorously stirring; (vi) placing the test tube in the magnetic support and removing the supernatant; (vii) adding 1.25 ml lysis solution (B), vigorously stirring, placing the tube in the magnetic support and removing the supernatant; (viii) washing three times with 70% ethanol, by vigorously stirring and removing the supernatant at each step; (ix) allowing the ethanol to evaporate; and (x) adding 200 µl water, vigorously stirring, placing the test tube in the magnetic support and collecting the DNA. The DNA extracted or frozen (preferably just extracted) is subjected to qPCR by using a commercial thermocycler for real-time PCR and PCR kinetics, preferably the Rotor-Gene 6000 (Corbett Research Ltd., Australia). The amplification reaction provides the use of a volume equal to 10-50 µl (preferably 12.5 µl) of a commercial master mix (preferably, Rotor-Gene SYBR Green RT-PCR Kit), a volume equal to 0.1-1 µl (preferably 0.75 µl) of each primer at the concentration of 10-100 µM (preferably 100 µM) (Qiagen, Italy), and a volume of 0.1-10 µl DNA (preferably 1 µl) having a concentration of 1-600 ng/µl. It was observed that the primer sequences must be such to selectively amplify a short sequence (< 300 bp) of the lactic acid bacteria genome. The following primer pair is employed: SKfw (5'-GGGGATAACAYYTGGAAACAG-3') (SEQ. ID NO. 1) and SKrw (5'-CTCGGCTACGTATCATTGTCTTG-3') (SEQ. ID NO. 2) amplifying a region of about 178 bp of 16S rRNA of the lactic acid bacteria. The amplification cycle, carried out on a reaction mixture of 15-50 µl or multiples (preferably 25 µl), provides a first denaturation step of the DNA at 80-100°C (preferably 95°C) for a variable time between 5 seconds and 20 minutes (preferably 10 minutes), followed by a number of amplification cycles variable between 10 and 50 (preferably 35), constituted by: (i) denaturation at 80-100°C (preferably 95°C) for a variable time between 5 seconds and 20 minutes (preferably 10 seconds); (ii) annealing at 45-65°C (preferably 55°C) for a variable time between 5 seconds and 10 minutes (preferably 30 seconds); and (iii) elongation at 65-80°C (preferably 72°C) for a variable time between 5 seconds and 15 minutes (preferably 30 seconds). To complete the amplification, the "melting curve" is carried out at a variable temperature ranging from 55 to 95°C, preferably between 60 and 95°C. The 1°C increment is preferred. The quantification is carried out by using specific software for the quantitative PCR, preferably by using the Rotor-Gene 6000 Series Software, version 1.7 and automatically or manually setting the "threshold" to a variable value between 0.1 and 1 (preferably a 0.4). The result of the analysis is the value of the threshold cycle (C_{T}).

### Examples

### Example 1: Evaluation of the primer specificity

The primer pair designed to selectively amplify 16S rRNA gene of the lactic acid bacteria belonging to the genera more frequently isolated from sourdough (e.g. *Enterococcus*, *Lactobacillus*, *Lactococcus*, *Leuconostoc*, *Pediococcus* and *Weissella*) has been assayed on the DNA directly extracted from 58 biotypes of lactic acid bacteria belonging to the above mentioned genera and 5 biotypes of bacteria recognized as contaminants of the flour (5) (Table 1). In particular, 12.5 µl Master Mix (Qiagen, Italy), 10 µl RNase free water, 0.75 µl per each primer (SKfw/SKrw), at the concentration of 100 µM, and 1 µl DNA directly extracted from the pure cultures of the 63 biotypes of lactic acid bacteria and contaminants of the flour, have been used.

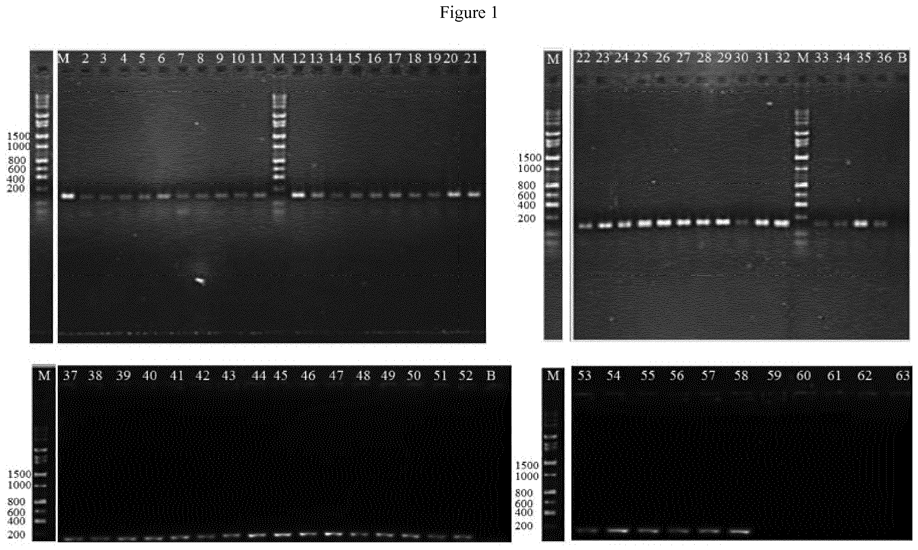

The PCR cycle included the denaturation of the DNA at 95°C for 10 minutes, followed by 35 cycles of: (i) denaturation of the DNA at 95°C for 10 seconds; (ii) annealing at 55°C for 30 seconds; and (iii) elongation at 72°C for 30 seconds. To complete the amplification, there has been a further elongation step at 72°C for 7 minutes. A volume of 5 µl each amplified DNA has been loaded on agarose gel (2% w/vol) and subjected to electrophoresis (90 mV for 50 minutes). Only the DNAs of the 38 species of considered lactic acid bacteria (Table 1) have been correctly amplified, and produce a common amplicon of about 178 bp (Figure 1). On the contrary, none of the biotypes of the bacteria that are contaminants of the flour showed an amplicon as a result of the amplification (Figure 1).

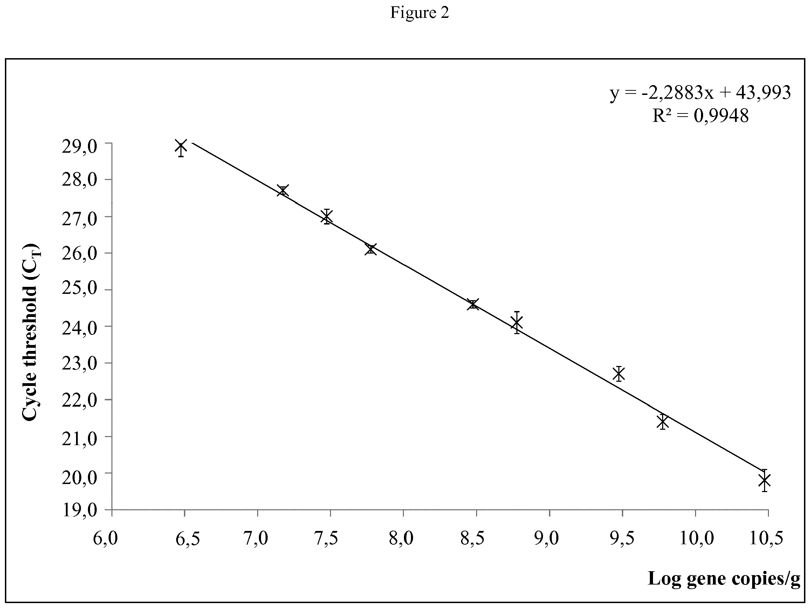

With the purpose of carrying out a quantification of the cellular density of the lactic acid bacteria present in the bread starting from the concentration of DNA related to them, without however neglecting the matrix-effect of the same, a standard curve has been generated by considering breads containing different cellular densities (about 6.0 - 9.0 log cfu/g) of a pure culture *of L. plantarum* (strain type WCFS1). After cooking, the breads have been subjected to DNA extraction and qPCR. The C_{T} values have been correlated to the number of copies of the 16S rRNA gene and a linear calibration curve, with a correlation coefficient (R²) of 0.9948 has been obtained (Table 2 and Figure 2).

In Figure 1: M, Standard (ladder) (Sigma); Lines 1 and 2, *Lactobacillus fermentum* ATCC 14931 and F1; Lane 3, *Weissella confusa* DSM 20196; Lanes 4 and 5, *Pediococcus pentosaceus* PP5 and PP10; Lane 6, *Lactobacillus curvatus* ATCC 25601; Lane 7, *Leuonostoc citreum* 22A; Lanes 8 and 9, *Weissella cibaria* 5S, 7S; Lane 10, *Lactobacillus pentosus* ATCC 8041; Lanes 11 and 12, *Lactobacillus amylovarus* L_amy and ATCC 33620; Lanes 13 - 18, *Lactobacillus plantarum* DC400, POM1, RED3, 1M, 5-5 and 3DM; Lane 19, *Lactococcus lactis* 10_{γ}, Lane 20, *Lactobacillus delbrueckii* B15Z; Lanes 21 - 23, *Lactobacillus rossiae* DSM 18514, LB5 and 8-5; Lanes 24 - 29, *Lactobacillus sanfranciscensis* 13, 7A, A1, E21, E14 and 274; Lane 30, *Lactobacillus brevis* LB4; Lanes 31 and 32, *Enterococcus faecalis* ATCC 23655 and 40; Lanes 33 and 34, *Enterococcus faecium* DSM 20477 and 22; Lane 35, *Lactobacillus hilgardii* ATCC 8290; Lane 36, *Lactobacillus hammesii* ATCC 23074; Lane 37, *L. acidifarinae* DSM 19394; Lane 38, *L. amylolyticus;* Lane 39, *L. frumenti* DSM 13145; Lane 40, *L. namurensis* DSM 19117; Lane 41, *L. nantensis* DSM 16982; Lane 42, *L. pontis* DSM 8475; Lane 43, *L. panis* DSM 6035; Lane 44, *L. crispatus* DSM 20584; Lane 45, *L. farciminis* DSM 20184; Lane 46, *Leuc. mesenteroides* subsp. *cremoris;* Lane 47, *Leuc. mesenteroides* subsp. *dextranicum* DSM 46216; Lanes 48 and 49, *Leuc. mesenteroides* subsp. *mesenteroides* DSM 20343 and LM5; Lane 50, *L. alimentarius;* Lane 51, *L. casei* ATCC-393; Lane 52, *L. paralimentarius* DSM 13238; Lane 53, *L. acidophilus* ATCC-4356; Lane 54, *L. kunkeei* LKA; Lane 55, *L. kunkeei* LKB; Lane 56, *P. acidilactici* DSM 20238; Lane 57, *L. helveticus* ATCC 15009; Lane 58, *L. reuteri* ATCC-23272; Lane 59, *Serratia marcescens;* Lane 60, *Staphylococcus capitis* Sc1; Lane 61, *Bacillus megaterium* Bm1; Lane 62, *Escherichia hermannii* Eh1; Lane 63, *Pantoea agglomerans* Pa1; B, negative control. The numbers on the left denote the amplicon dimension expressed in base pairs (bp).

**Table 1. Biotypes of lactic acid bacteria and contaminants of the flour, employed for the evaluation of the primer specificity.**

| **Strain** | **Collection Body*** | **Strain** | **Collection Body *** |
|---|---|---|---|
| Lactic acid bacteria | | | |
| *Lactobacillus fermentum* ATCC 14931 | ATCC | *L. plantarum* 4-4, 3-3, 1M, 5-5, 3DM | DiSSPA |
| *L. fermentum* F 1 | DiSSPA | *Lactobacillus delbrueckii* B15Z | DiSSPA |
| *Weissella confusa* DSM 20196 | DSM | *Lactobacillus rossiae* DSM 18514 | DSMZ |
| *Pediococcus pentosaceus* PP10, PP5 | DiSSPA | *L. rossiae* LB5, 8-5 | DiSSPA |
| *Lactobacillus curvatus* ATCC 25601 | ATCC | *Lactobacillus sanfranciscensis* 7A, 13, A1, E14, E21, 274 | DiSSPA |
| *Leuonostoc citreum* 22A | DiSSPA | *Lactobacillus brevis* LB4 | DiSSPA |
| *Weissella cibaria* 5S, 7S | DiSSPA | *Lactobacillus hilgardii* ATCC 8290 | ATCC |
| *Lactobacillus pentosus* ATCC 8041 | ATCC | *Lactobacillus hammesii* BCCM/LMG 23074 | BCCM |
| *Lactobacillus amylovorus* L_amy | DiSSPA | *Enterococcus faecium 22* | ISPA-CNR |
| *L. amylovorus* ATCC 33620 | ATCC | *Enterococcus faecium* DSM 20477 | DSMZ |
| *Lactococcus lactis* 10γ | DiSSPA | *Enterococcus faecalis* ATCC 23655 | ATCC |
| *Lactobacillus plantarum* DC400 | DiSSPA | *Enterococcus faecalis* 40 | ISPA-CNR |
| *L. acidifarinae* DSM 19394 | DSMZ | *Leuc. mesenteroides* subsp. *mesenteroides* DSM 20343 | DSMZ |
| *L. frumenti* DSM 13145 | DSMZ | *L. alimentarius* ATCC 29643 | ATCC |
| *L. kunkeei* LKA | DiSSPA | *L. casei* ATCC-393 | ATCC |
| *L. kunkeei* LKB | DiSSPA | *L. paralimentarius* DSM 13238 | DSMZ |
| *L. nantensis* DSM 16982 | DSMZ | *P. acidilactici* DSM 20238 | DSMZ |
| *L. panis* DSM 6035 | DSMZ | *L. acidophilus* ATCC-4356 | ATCC |
| *L. pontis* DSM 8475 | DSMZ | *L. amylolyticus* DSM 11664 | DSMZ |
| *L. reuteri* ATCC-23272 | ATCC | *L. crispatus* DSM 20584 | DSMZ |
| *L. helveticus* ATCC 15009 | ATCC | *Leuc. mesenteroides* subsp. *cremoris* DSM 20346 | DSMZ |
| *L. farciminis* DSM 20184 | DSMZ | *Leuc. mesenteroides* subsp. *dextranicum* DSM 46216 | DSMZ |
| *Leuc. mesenteroides* subsp. *mesenteroides* LM5 | DiSSPA | *L. namurensis* DSM 19117 | DSMZ |

| Contaminants of the flour | | | |
|---|---|---|---|
| *Se. marcescens* Sm1 | DISSPA | *St. capitis* Sc1 | DiSSPA |
| *B. megaterium* Bm1 | DiSSPA | *Pa. agglomerans* Pa1 | DiSSPA |
| *Esch. hermannii* Eh1 | DiSSPA | | |

| | | | |
|---|---|---|---|
| * BCCM/LMG, Belgian Coordinated Collections of Microorganisms; Di.S.S.P.A., Dipartimento di Scienze del Suolo, della Pianta e degli Alimenti, Aldo Moro, Italy; ATCC, American Type Culture Collection; DSMZ, Deutsche Sammlung von Mikroorganismen and Zellkulturen; ISPA-CNR, Istituto di Scienze degli Alimenti - Institute of Sciences of Food Production - Consiglio Nazionale delle Ricerche (CNR), Bari, personal collection of the Dr. P. Lavermicocca. | | | |

**Table 2. Cellular density (log cfu/g), cycle threshold (C_{T}), and number of copies of the 16S rRNA gene (log gene copies/g) of breads containing serial dilutions of L. plantarum WCFS1 used to generate the calibration curve.**

| **Cellular density (log cfu/g)** | **Cycle threshold (C_{T})** | **Number of copies of the gene (log gene copies/g)** |
|---|---|---|
| 5.8 ± 0.1 | 28.9 ± 0.2 | 6.5 ± 0.2 |
| 6.5 ± 0.2 | 27.7 ± 0.1 | 7.2 ± 0.1 |
| 6.8 ± 0.1 | 27.0 ± 0.2 | 7.5 ± 0.2 |
| 7.1 ± 0.2 | 26.1 ± 0.1 | 7.8 ± 0.1 |
| 7.8 ± 0.3 | 24.6 ± 0.1 | 8.5 ± 0.1 |
| 8.1 ± 0.1 | 24.1 ± 0.3 | 8.8 ± 0.3 |
| 8.8 ± 0.1 | 22.7 ± 0.2 | 9.5 ± 0.2 |
| 9.1 ± 0.2 | 21.4 ± 0.2 | 9.8 ± 0.2 |
| 9.8 ± 0.3 | 19.8 ± 0.3 | 10.5 ± 0.3 |

### Example 2: Definition of the detection limit.

In order to define the detection limit able to ensure differentiating between bread with natural leavening and bread produced with brewer's yeast only, 65 breads (lab breads) have been prepared at the pilot plant of DiSSPA. Table 3 is summarizing the ingredients, technological parameters and the cellular density of the lactic acid bacteria. The breads with natural leavening have been produced by using sourdoughs prepared in the lab (TA and TD) or purchased at handicraft bakeries (MTA, ALB, ALA, CG, V, and BA). Various percentages (10 - 100%, w/w) of sourdough have been used, and the duration of the fermentation varied from 1.5 to 4 hours. The addition (1.5%, w/w) of brewer's yeast has been carried out only for breads the leavening of which had short duration (1.5 hours). The sourdoughs coded as V, ALA and BA have been diluted 1:10 with flour and water (DY 180). These sourdoughs have been coded as Vd, ALAd and BAd. The above mentioned conditions allowed reaching cellular densities of lactic acid bacteria variable between 6.0 ± 0.2 and 8.7 ± 0.1 log cfu/g, before the dough is subjected to cooking (Table 3). In order to simulate the chemical leavening, two breads have been produced the dough of which has been chemically acidified by the use of a mixture of lactic and acetic acid until reaching a pH value equal to about 4.0. After the production, the breads have been subjected to DNA extraction following the protocol mentioned in the "Methods" section and the DNA has been used for the qPCR reactions. In particular, 12.5 µl Rotor-Gene SYBR Green RT-PCR master mix, 0.75 µl of each primer (SKfw/SKrw), at a concentration of 100 µM, 10 µl RNase free water and 1 µl DNA have been employed for the amplification.

The amplification cycle has been carried out on a reaction mixture of 25 µl in Rotor-Gene 6000 (Corbett Research Ltd., Australia), and included a first denaturation step of the DNA at 95°C for 10 minutes, followed by 35 cycles of: (i) denaturation at 95°C for 10 seconds; (ii) annealing at 55°C for 30 seconds; and (iii) elongation at 72°C for 30 seconds. At the end of the amplification the "melting curve" has been carried out at a temperature range of 60 - 95°C, with 1°C increments. The quantification has been carried out by using the Rotor-Gene 6000 Series Software, version 1.7, and manually setting the "threshold" at a value of 0.4.

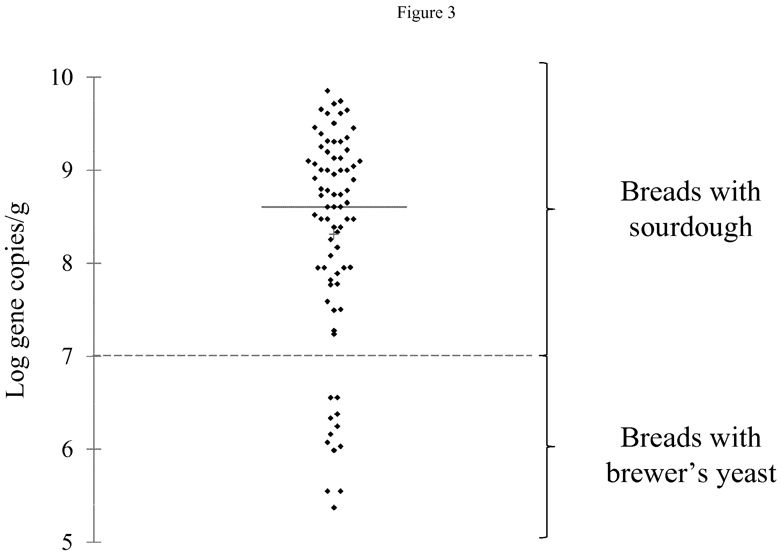

Table 4 and Figure 3 show the results in terms of CT and number of copies of the 16S rRNA gene of lab breads, used to define the detection limit able to differentiate between bread with natural leavening and bread obtained with brewer's yeast. The number of copies of the 16S rRNA gene (log gene copies/g) of the breads with natural leavening varied (7.2 ± 0.3 - 9.9 ± 0.3 log gene copies/g) significantly (p <0.05) as a function of the cellular density of the lactic acid bacteria (6.0 ± 0.2 - 8.7 ± 0.1 log cfu/g). When the breads produced with brewer's yeast (density of lactic acid bacteria variable from 2.5 ± 0.2 to 4.4 ± 0.3 log cfu/g) have been analyzed, the number of copies of the 16S rRNA gene was between 5.5 ± 0.7 to 6.6 ± 0.1 (log gene copies/g) (Table 4 and Figure 3). As showed by the scatterplot (Figure 3) based on the values of number of copies of the 16S rRNA gene, 100% of the lab breads with natural leavening were distributed above the value of number of copies of the 16S rRNA gene of 7 log gene copies/g, whereas 100% of the breads obtained with brewer's yeast have grouped below the same value. The bread subjected to chemical acidification showed values of number of copies of the 16S rRNA gene < 7 log gene copies/g, highlighting that there isn't any correlation between the bread acidity and the C_{T} values. Based on the above mentioned results, it is possible to define the value 7 log gene copies/g as the differentiating limit between breads with natural leavening and breads obtained employing brewer's yeast only.

Figure 3 shows the Scatterplot based on the value of number of copies of the 16S rRNA gene of lab breads: Average (+), median (red line) and differentiating limit (dashed line) are shown.

**Table 3. Ingredients, technological parameters and cellular density of lactic acid bacteria of the lab breads with natural leavening or with brewer's yeast only.**

| **Code** | **Flour (g)** | **Water (g)** | **Sourdough (g)** | **Lactic acid bacteria (log cfu/g)** | **Brewer's yeast (g)** | **Leavening time (h)** |
|---|---|---|---|---|---|---|
| | | Sourdough TA | | | | |
| TA1 | 112.5 | 67.5 | 20 | 7.9 ± 0.1 | 3 | 1.5 |
| TA2 | 100 | 60 | 40 | 8.2 ± 0.2 | | |
| TA3 | 87.5 | 52.5 | 60 | 8.4 ± 0.1 | | |
| TA4 | 75 | 45 | 80 | 8.5 ± 0.3 | | |
| TA5 | 100 | 60 | 40 | 8.2 ± 0.2 | - | 4 |
| TA6 | 87.5 | 52.5 | 60 | 8.4 ± 0.1 | | |
| TA7 | 75 | 45 | 80 | 8.5 ± 0.3 | | |

| | | Sourdough TD | | | | |
|---|---|---|---|---|---|---|
| TD1 | 112.5 | 67.5 | 20 | 8.1 ± 0.2 | 3 | 1.5 |
| TD2 | 100 | 60 | 40 | 8.3 ± 0.2 | | |
| TD3 | 87.5 | 52.5 | 60 | 8.5 ± 0.3 | | |
| TD4 | 75 | 45 | 80 | 8.7 ± 0.1 | | |
| TD5 | 100 | 60 | 40 | 8.3 ± 0.2 | - | 4 |
| TD6 | 87.5 | 52.5 | 60 | 8.5 ± 0.3 | | |
| TD7 | 75 | 45 | 80 | 8.7 ± 0.1 | | |

| | | Sourdough MTA | | | | |
|---|---|---|---|---|---|---|
| MTA1 | 112.5 | 67.5 | 20 | 7.7 ± 0.2 | 3 | 1.5 |
| MTA2 | 100 | 60 | 40 | 8.2 ± 0.2 | | |
| MTA3 | 87.5 | 52.5 | 60 | 8.3 ± 0.1 | | |
| MTA4 | 75 | 45 | 80 | 8.6 ± 0.3 | | |
| MTA5 | 100 | 60 | 40 | 8.2 ± 0.2 | - | 4 |
| MTA6 | 87.5 | 52.5 | 60 | 8.3 ± 0.1 | | |
| MTA7 | 75 | 45 | 80 | 8.6 ± 0.3 | | |

| Sourdough ALB | | | | | | |
|---|---|---|---|---|---|---|
| ALB1 | 112.5 | 67.5 | 20 | 7.8 ± 0.3 | 3 | 1.5 |
| ALB2 | 100 | 60 | 40 | 8.1 ± 0.3 | | |
| ALB3 | 87.5 | 52.5 | 60 | 8.4 ± 0.1 | | |
| ALB4 | 75 | 45 | 80 | 8.6 ± 0.2 | | |
| ALB5 | 100 | 60 | 40 | 8.1 ± 0.3 | - | 4 |
| ALB6 | 87.5 | 52.5 | 60 | 8.4 ± 0.1 | | |
| ALB7 | 75 | 45 | 80 | 8.6 ± 0.2 | | |

| Sourdough CG | | | | | | |
|---|---|---|---|---|---|---|
| CG1 | 112.5 | 67.5 | 20 | 7.7 ± 0.3 | 3 | 1.5 |
| CG2 | 100 | 60 | 40 | 8.2 ± 0.3 | | |
| CG3 | 87.5 | 52.5 | 60 | 8.5 ± 0.1 | | |
| CG4 | 75 | 45 | 80 | 8.5 ± 0.1 | | |
| CG5 | 100 | 60 | 40 | 8.2 ± 0.3 | - | 4 |
| CG6 | 87.5 | 52.5 | 60 | 8.5 ± 0.1 | | |
| CG7 | 75 | 45 | 80 | 8.5 ± 0.1 | | |

| Sourdough V | | | | | | |
|---|---|---|---|---|---|---|
| V1 | 44.4 | 135.6 | 20 | 7.3 ± 0.1 | 3 | 1.5 |
| V2 | 83.3 | 66.7 | 50 | 7.6 ± 0.2 | | |
| V3 | 55.5 | 44.4 | 100 | 7.8 ± 0.2 | | |
| V4 | 27.7 | 22.3 | 150 | 7.9 ± 0.3 | | |
| V5 | - | - | 200 | 8.3 ± 0.1 | | |

| Sourdough V*d* | | | | | | |
|---|---|---|---|---|---|---|
| Vd1 | 44.4 | 135.6 | 20 | 6.0 ± 0.2 | 3 | 1.5 |
| Vd2 | 83.3 | 66.7 | 50 | 6.5 ± 0.1 | | |
| Vd3 | 55.5 | 44.4 | 100 | 6.8 ± 0.3 | | |
| Vd4 | 27.7 | 22.3 | 150 | 6.9 ± 0.2 | | |
| Vd5 | - | - | 200 | 7.3 ± 0.1 | | |

| Sourdough ALA | | | | | | |
|---|---|---|---|---|---|---|
| ALA1 | 44.4 | 135.6 | 20 | 7.3 ± 0.1 | 3 | 1.5 |
| ALA2 | 83.3 | 66.7 | 50 | 7.6 ± 0.3 | | |
| ALA3 | 55.5 | 44.4 | 100 | 7.8 ± 0.2 | | |
| ALA4 | 27.7 | 22.3 | 150 | 7.9 ± 0.1 | | |
| ALA5 | - | - | 200 | 8.3 ± 0.2 | | |
| Sourdough ALAd | | | | | | |
| ALAd1 | 44.4 | 135.6 | 20 | 6.1 ± 0.2 | 3 | 1.5 |
| ALAd2 | 83.3 | 66.7 | 50 | 6.6 ± 0.2 | | |
| ALAd3 | 55.5 | 44.4 | 100 | 6.9 ± 0.3 | | |
| ALAd4 | 27.7 | 22.3 | 150 | 7.0 ± 0.1 | | |
| ALAd5 | - | - | 200 | 7.2 ± 0.2 | | |
| Sourdough BA | | | | | | |
| BA1 | 44.4 | 135.6 | 20 | 7.4 ± 0.2 | 3 | 1.5 |
| BA2 | 83.3 | 66.7 | 50 | 7.6 ± 0.1 | | |
| BA3 | 55.5 | 44.4 | 100 | 7.8 ± 0.2 | | |
| BA4 | 27.7 | 22.3 | 150 | 8.0 ± 0.1 | | |
| BA5 | - | - | 200 | 8.4 ± 0.3 | | |
| Sourdough BAd | | | | | | |
| BAd1 | 44.4 | 135.6 | 20 | 6.0 ± 0.3 | 3 | 1.5 |
| BAd2 | 83.3 | 66.7 | 50 | 6.5 ± 0.1 | | |
| BAd3 | 55.5 | 44.4 | 100 | 6.9 ± 0.3 | | |
| BAd4 | 27.7 | 22.3 | 150 | 7.1 ± 0.2 | | |
| BAd5 | - | - | 200 | 7.3 ± 0.1 | | |
| Bread with brewer's yeast | | | | | | |
| BY1 | 111,11^{a} | 88.9 | - | 3.1 ± 0.1 | 4 | 1.5 |
| BY2 | | | | 3.0 ± 0.3 | | |
| BY3 | | | | 3.0 ± 0.1 2.5 ± | | |
| BY4 | | | | 0.3 | | |
| BY5 | | | | 2.5 ± 0.2 | | |
| BY6 | | | | 3.7 ± 0.2 | | |
| BY7 | 111,11^{b} | 88.9 | - | 3.1 ± 0.1 | 4 | 1.5 |
| BY8 | | | | 4.4 ± 0.3 | | |
| BY9 | | | | 2.6 ± 0.2 | | |
| BY10 | | | | 3.7 ± 0.3 | | |
| BY11 | | | | 3.5 ± 0.1 | | |
| BY12 | | | | 4.4 ± 0.1 | | |

| | | | | | | |
|---|---|---|---|---|---|---|
| ^{a}*Triticum aestivum;* ^{b}*Triticum durum* | | | | | | |

**Table 4. Cycle threshold (C_{T}) and number of copies of the 16S rRNA gene (log gene copies/g) of lab breads obtained by using the sourdough or brewer's yeast as a leavening agent and which are used to define the detection limit of the qPCR.**

| **Code** | **Cycle threshold (C_{T})** | **N umber of copies of the gene (log gene copies/g)** |
|---|---|---|
| Bread with sourdough | | |
| Vd1 | 29.5 ± 0.2 | 7.3 |
| Vd2 | 27.3 ± 0.5 | 7.6 |
| Vd3 | 26.6 ± 0.3 | 7.8 |
| Vd4 | 25.6 ± 0.3 | 7.9 |
| Vd5 | 24.0 ± 0.2 | 7.5 |
| V1 | 23.8 ± 0.2 | 8.0 |
| V2 | 22.6 ± 0.3 | 9.2 |
| V3 | 22.8 ± 0.2 | 9.5 |
| V4 | 22.0 ± 0.3 | 9.9 |
| V5 | 21.8 ± 0.2 | 9.7 |
| ALAd1 | 29.4 ± 0.4 | 7.5 |
| ALAd2 | 27.4 ± 0.3 | 8.3 |
| ALAd3 | 26.4 ± 0.2 | 8.5 |
| ALAd4 | 25.1 ± 0.9 | 8.8 |
| ALAd5 | 23.6 ± 0.4 | 8.9 |
| ALA1 | 23.2 ± 0.5 | 9.5 |
| ALA2 | 22.8 ± 0.4 | 9.3 |
| ALA3 | 20.9 ± 0.4 | 9.3 |
| ALA4 | 21.4 ± 0.6 | 9.7 |
| ALA5 | 20.4 ± 0.3 | 9.6 |
| BA*d*1 | 29.8 ± 0.3 | 7.2 |
| BA*d*2 | 27.5 ± 0.3 | 8.3 |
| BA*d*3 | 26.5 ± 0.3 | 8.5 |
| BA*d*4 | 25.2 ± 0.3 | 8.7 |
| BA*d*5 | 24.1 ± 0.4 | 9.0 |
| BA1 | 24.7 ± 0.8 | 9.1 |
| BA2 | 23.2 ± 0.4 | 9.1 |
| BA3 | 23.4 ± 0.2 | 9.1 |
| BA4 | 22.3 ± 0.3 | 9.5 |
| BA5 | 21.9 ± 0.3 | 9.6 |
| CG1 | 26.1 ± 0.3 | 7.8 |
| CG2 | 24.0 ± 0.5 | 8.7 |
| CG3 | 24.3 ± 0.6 | 8.6 |
| CG4 | 22.6 ± 0.1 | 9.3 |
| CG5 | 23.4 ± 0.9 | 9.0 |
| CG6 | 23.4 ± 0.2 | 9.0 |
| CG7 | 22.9 ± 0.4 | 9.2 |
| MTA1 | 26.2 ± 0.3 | 7.8 |
| MTA2 | 25.3 ± 0.9 | 8.2 |
| MTA3 | 25.5 ± 0.1 | 8.1 |
| MTA4 | 24.6 ± 0.7 | 8.5 |
| MTA5 | 25.8 ± 0.1 | 8.0 |
| MTA6 | 25.8 ± 0.4 | 8.0 |
| MTA7 | 25.8 ± 0.3 | 8.0 |
| TD1 | 24.3 ± 1.0 | 8.6 |
| TD2 | 23.9 ± 0.4 | 8.8 |
| TD3 | 24.3 ± 0.5 | 8.6 |
| TD4 | 23.1 ± 0.1 | 9.1 |
| TD5 | 24.6 ± 0.4 | 8.5 |
| TD6 | 23.5 ± 0.6 | 9.0 |
| TD7 | 23.1 ± 1.5 | 9.1 |
| TA1 | 22.7 ± 1.3 | 9.3 |
| TA2 | 21.9 ± 0.6 | 9.7 |
| TA3 | 22.0 ± 0.8 | 9.6 |
| TA4 | 23.9 ± 0.6 | 8.8 |
| TA5 | 24.8 ± 0.5 | 8.4 |
| TA6 | 24.8 ± 0.9 | 8.4 |
| TA7 | 23.6 ± 0.0 | 8.9 |
| ALB1 | 23.3 ± 0.6 | 9.0 |
| ALB2 | 24.6 ± 1.5 | 8.5 |
| ALB3 | 23.4 ± 0.4 | 9.0 |
| ALB4 | 22.7 ± 0.1 | 9.3 |
| ALB5 | 24.2 ± 0.6 | 8.6 |
| ALB6 | 24.0 ± 0.5 | 8.7 |
| ALB7 | 22.5 ± 0.1 | 9.4 |

| Bread with brewer's yeast | | |
|---|---|---|
| BY1 | 29.6 ± 0.7 | 6.6 |
| BY2 | 29.4 ± 0.4 | 6.4 |
| BY3 | 29.8 ± 1.4 | 6.6 |
| BY4 | 30.1 ± 1.1 | 6.1 |
| BY5 | 29.5 ± 0.5 | 6.3 |
| BY6 | 30.2 ± 0.4 | 6.0 |
| BY7 | 29.9 ± 0.7 | 6.2 |
| BY8 | 31.7 ± 2.6 | 5.4 |
| BY9 | 30.3 ± 1.3 | 6.0 |
| BY10 | 31.3 ± 0.6 | 5.5 |
| BY11 | 31.3 ± 1.4 | 5.5 |
| BY12 | 29.7 ± 0.5 | 6.2 |

### Example 3: Determination of the efficiency of the qPCR protocol.

The efficiency of the qPCR protocol, optimized according to Example 2, has been further evaluated on 93 breads purchased at bakeries and supermarkets (Table 5). The breads have been subjected to extraction of the total DNA by the method mentioned in the "Methods" section. The DNA extracted has been employed according to the qPCR protocol. In particular, 12.5 µl Rotor-Gene SYBR Green RT-PCR master mix, 0.75 µl of each primer (SKfw/SKrw), at a concentration of 100 µM, 10 µl RNase free water and 1 µl DNA extracted from the breads have been employed for the amplification. The amplification cycle has been carried out on a reaction mixture of 25 µl in Rotor-Gene 6000 (Corbett Research Ltd., Australia), and included a first denaturation step of the DNA at 95°C for 10 minutes, followed by 35 cycles of: (i) denaturation at 95°C for 10 seconds; (ii) annealing at 55°C for 30 seconds; and (iii) elongation at 72°C for 30 seconds. To complete the amplification, the "melting curve" has been carried out in a temperature range of 60 - 95°C, with 1°C increments. The quantification has been carried out by using the Rotor-Gene 6000 Series Software, version 1.7, by manually setting the "threshold" at a value of 0.4 and the determination of the number of copies of the 16S rRNA gene by using the equation of the calibration line depicted in Figure 2.

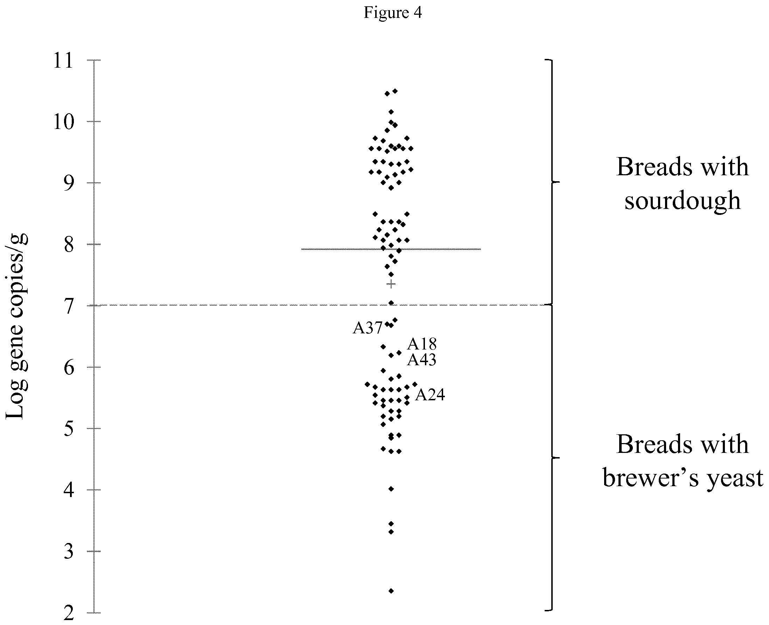

93% of the samples declared as produced with natural leavening showed a value of number of copies of the 16S rRNA gene > 7 log gene copies/g, whereas 100% of those sold as produced with brewer's yeast have been grouped below the threshold value (7 log gene copies/g) (Table 6 and Figure 4). The only exceptions have been the not packaged breads sold with natural leavening with the codes A18, A24, A37, and A43.

They showed values of 5.5 ± 0.2, 6.2 ± 0.1, 6.2 ± 0.2, and 6.7 ± 0.3 log gene copies/g, respectively. These breads, versus all the other sold as being with natural leavening, had biochemical characteristics similar to those detected in the breads obtained by brewer's yeast. In order to simulate the industrial production of bread with natural leavening, 21 breads (P1-P21) containing commercial dehydrated sourdough (Brand A - Brand G), with different inoculum levels, have been produced at the pilot plant of DiSSPA. In particular, each dehydrated sourdough has been employed at 8 % [w/w; based on the weight of the flour], according to what depicted on the label by the producer. Furthermore, with the purpose of defining the sensitivity of the method, two extreme percentages: 4 % [w/w, based on the weight of the flour] and 20 % [w/w, based on the weight of the dough] have been employed in the formulations (Table 7).

Table 8 depicts the results relative to the breads containing dehydrated sourdough only, as leavening agent and which have been produced at the pilot plant of DiSSPA. All of the breads showed a number of copies of the 16S rRNA gene > 7 log gene copies/g when produced according to the indications depicted on the label of the employed dehydrated sourdough. The number of copies of the 16S rRNA gene varied as a function of the percentage of dehydrated mother yeast employed in the preparation of the bread (Table 8). Such results highlighted a good response of the method, and the presence of a concentration of DNA of lactic acid bacteria in the dehydrated sourdough compatible with the natural leavening.

Figure 4 shows the Scatterplot based on the values of number of copies of the 16S rRNA gene (log gene copies/g) of breads purchased at the bakeries and supermarkets: Average (+), median (red line) and differentiating limit (dashed line) are shown. The brands coded as A18, A24, A37 and A43 are produced without packaging, without label and sold as breads with natural leavening.

**Table 5. Description of the breads purchased at bakeries or supermarkets.**

| **Code** | **Description** | **Commercial source** |
|---|---|---|
| Bread with sourdough | | |
| A | Bread with natural leavening with "Senatore Cappelli" wheat | Handcrafted; Italy |
| B | Bread with natural leavening with spelt | Handcrafted; Italy |
| C | Bread with natural leavening with almonds | Handcrafted; Italy |
| D | Bread with natural leavening with "Khorasan" wheat | Handcrafted; Italy |
| E | Bread with wholemeal flour with natural leavening | Handcrafted; Italy |
| I | Greek bread, with sourdough | Handcrafted; Italy |
| L | Bread with natural leavening with "Abbondanza" wheat | Handcrafted; Italy |
| M | Bread with natural leavening with turmeric | Handcrafted; Italy |
| N | Bread with natural leavening with fruit | Handcrafted; Italy |
| O | Organic rye bread with sourdough | Handcrafted; Italy |
| P | Wheat bread with sourdough | Handcrafted; Italy |
| Q | Focaccia with sourdough | Handcrafted; Italy |
| T | Wheat bread with sourdough | Handcrafted; Italy |
| U | Wheat bread with sourdough | Supermarket; Italy |
| V | Wheat bread with fermented bran | Supermarket; Italy |
| Z | Bread with fermented bran & germ | Supermarket; Italy |
| A1 | Wheat bread with sourdough | Handcrafted; Italy |
| A2 | Wheat bread with sourdough | Handcrafted; Italy |
| A3 | Wheat bread with sourdough | Handcrafted; Italy |
| A4 | Wheat croissant with sourdough | Handcrafted; Italy |
| A5 | Wheat bread with sourdough | Supermarket; Italy |
| A6 | Wheat bread with fermented bran | Supermarket; Italy |
| A7 | Bread with fermented bran & germ | Supermarket; Italy |
| A8 | Bread "Altamura" type | Handcrafted; Italy |
| A9 | Bread with wheat sourdough | Supermarket; Italy |
| A11 | Sliced bread with wheat sourdough | Supermarket; Italy |
| A16 | Bread with wheat sourdough | Supermarket; Italy |
| A17 | Bread with wheat sourdough | Handcrafted; Italy |
| A18 | Bread with wheat sourdough | Handcrafted; Italy |
| A19 | Bread with liquid wheat sourdough | Supermarket; Italy |
| A21 | Bread with wheat sourdough | Handcrafted; Italy |
| A24 | Bread with wheat sourdough | Handcrafted; Italy |
| A26 | Bread with wheat sourdough | Handcrafted; Italy |
| A29 | Wheat sourdough, barley bread | Handcrafted; Italy |
| A31 | Wheat sourdough, buckwheat bread | Handcrafted; Italy |
| A32 | Sliced bread with wheat sourdough | Supermarket; Italy |
| A37 | Bread with wheat sourdough | Handcrafted; Italy |
| A40 | Bread with wheat sourdough | Supermarket; Italy |
| A41 | Bread with wheat sourdough | Supermarket; Italy |
| A42 | Bread with wheat sourdough | Supermarket; Italy |
| A43 | Bread with wheat sourdough | Supermarket; Italy |
| A44 | Bread with wheat sourdough | Handcrafted; Italy |
| A47 | "Altamura" bread | Handcrafted; Italy |
| A51 | Puccia of wheat with sourdough | Supermarket; Italy |
| A52 | Puccia of wheat with sourdough | Supermarket; Italy |
| 2 | Wheat bread with sourdough (100%) | Handcrafted; Ireland |
| 6 | Baguette | Supermarket; Ireland |
| 8 | Wheat sourdough, bread with 70% rye & 30% wheat | Handcrafted; Ireland |
| 11 | Wheat bread with sourdough | Handcrafted; Ireland |
| 12 | Wholemeal bread with sourdough (55%) | Handcrafted; Ireland |
| 13 | White bread with wheat sourdough (65%) | Handcrafted; Ireland |
| 14 | Rye bread, 100% sourdough | Handcrafted; Ireland |
| 15 | Rye bread with 40% sourdough | Handcrafted; Ireland |
| 26 | Bread with 60% rye & 40% wheat | Handcrafted; Ireland |
| 27 | Six cereals bread, with wheat sourdough | Handcrafted; Ireland |
| 28 | Bread 50% spelt & 50% rye | Handcrafted; Ireland |
| 29 | French loaf with wheat sourdough | Handcrafted; Ireland |
| 32 | "San Francisco" bread | Supermarket; Ireland |

| Breads with brewer's yeast | | |
|---|---|---|
| G | Barley bread | Supermarket; Italy |
| R | Sliced white bread | Supermarket; Italy |
| H | Rye bread | Supermarket; Italy |
| A10 | Wheat bread | Handcrafted; Italy |
| A12 | Wheat bread | Handcrafted; Italy |
| A13 | Wheat bread | Handcrafted; Italy |
| A14 | Wheat bread | Handcrafted; Italy |
| A15 | Wheat bread | Handcrafted; Italy |
| A20 | Wheat bread | Handcrafted; Italy |
| A22 | Wheat bread | Supermarket; Italy |
| A23 | Wheat bread | Supermarket; Italy |
| 1 | Wheat bread | Supermarket; Ireland |
| 4 | Rosetta made of wheat | Supermarket; Ireland |
| 5 | Sliced wheat bread | Supermarket; Ireland |
| 9 | Wheat bread | Handcrafted; Ireland |
| 10 | Wheat bread | Handcrafted; Ireland |
| 16 | Sliced wheat bread | Supermarket; Ireland |
| 18 | Wholemeal bread with high fiber content | Supermarket; Ireland |
| 19 | Wholemeal, multi cereals and grains, bread | Handcrafted; Ireland |
| 20 | Wholemeal bread | Handcrafted; Ireland |
| 22 | Wheat bread | Supermarket; Ireland |
| 23 | Wheat bread | Supermarket; Ireland |
| 30 | Sliced wheat bread | Supermarket; Ireland |
| 31 | Sliced wheat bread | Supermarket; Ireland |
| A25 | Wheat bread | Supermarket; Italy |
| A27 | Wheat bread | Supermarket; Italy |
| A28 | Wheat bread | Handcrafted; Italy |
| A30 | Sliced wheat bread | Supermarket; Italy |
| A33 | Sliced wheat bread | Supermarket; Italy |
| A34 | Sliced wheat bread | Supermarket; Italy |
| A35 | Kamut bread | Handcrafted; Italy |
| A48 | Wheat bread | Handcrafted; Italy |
| A49 | Wheat bread | Handcrafted; Italy |
| A50 | Wheat bread | Handcrafted; Italy |
| A45 | Wheat bread | Handcrafted; Italy |
| A46 | Wheat bread | Handcrafted; Italy |
| A39 | Wheat bread | Handcrafted; Italy |
| A38 | Wheat bread | Handcrafted; Italy |

**Table 6. Values of number of copies of the 16S rRNA gene (log gene copies/g) of breads with sourdough or brewer's yeast, purchased at the bakeries or supermarkets.**

| **Code** | **Cycle threshold (C_{T})** | **Number of copies of the gene (log gene copies/g)** |
|---|---|---|
| Bread with sourdough | | |
| A | 26.0 ± 0.3 | 7.9 |
| B | 22.1 ± 0.7 | 9.6 |
| C | 22.1 ± 0.5 | 9.6 |
| D | 22.2 ± 0.9 | 9.5 |
| E | 22.1 ± 0.2 | 9.6 |
| I | 22.7 ± 0.4 | 9.3 |
| L | 21.7 ± 0.3 | 9.7 |
| M | 22.6 ± 0.2 | 9.3 |
| N | 23.6 ± 0.4 | 8.9 |
| O | 20.7 ± 0.7 | 10.2 |
| P | 21.8 ± 0.4 | 9.7 |
| Q | 26.9 ± 0.3 | 7.5 |
| T | 23.0 ± 0.7 | 9.2 |
| U | 20.0 ± 0.4 | 10.5 |
| V | 19.9 ± 0.3 | 10.5 |
| Z | 22.9 ± 0.3 | 9.2 |
| A2 | 25.6 ± 0.3 | 8.1 |
| A3 | 22.6 ± 1.3 | 9.3 |
| A4 | 22.1 ± 0.9 | 9.6 |
| A5 | 22.0 ± 0.3 | 9.6 |
| A6 | 22.7 ± 1.0 | 9.3 |
| A7 | 21.4 ± 1.2 | 9.9 |
| A8 | 25.6 ± 0.3 | 8.1 |
| A9 | 24.9 ± 0.6 | 8.4 |
| A11 | 26.4 ± 0.3 | 7.7 |
| A16 | 23.4 ± 1.3 | 9.0 |
| A17 | 25.6 ± 0.1 | 8.1 |
| A18 | 29.9 ± 0.8 | 6.2 |
| A19 | 24.9 ± 0.7 | 8.4 |
| A21 | 25.4 ± 1.1 | 8.2 |
| A24 | 31.6 ± 1.5 | 5.5 |
| A26 | 25.8 ± 0.2 | 8.0 |
| A29 | 23.0 ± 0.4 | 9.2 |
| A31 | 23.0 ± 0.1 | 9.2 |
| A32 | 25.2 ± 0.7 | 8.2 |
| A37 | 28.8 ± 0.6 | 6.7 |
| A40 | 21.7 ± 0.4 | 9.7 |
| A41 | 23.2 ± 0.9 | 9.1 |
| A42 | 22.0 ± 0.1 | 9.6 |
| A43 | 30.0 ± 1.5 | 6.2 |
| A44 | 21.1 ± 0.4 | 10.0 |
| A47 | 24.9 ± 0.4 | 8.4 |
| A51 | 25.9 ± 0.6 | 7.9 |
| A52 | 25.5 ± 1.1 | 8.1 |
| 2 | 24.6 ± 0.3 | 8.5 |
| 6 | 26.2 ± 0.9 | 7.8 |
| 8 | 25.2 ± 0.2 | 8.2 |
| 11 | 28.0 ± 0.3 | 7.0 |
| 12 | 25.0 ± 0.3 | 8.3 |
| 14 | 22.6 ± 0.2 | 8.6 |
| 15 | 24.6 ± 0.3 | 9.3 |
| 26 | 23.4 ± 0.6 | 8.5 |
| 27 | 22.1 ± 0.4 | 9.0 |
| 28 | 26.6 ± 0.9 | 9.6 |
| 29 | 23.1 ± 0.6 | 7.6 |
| 32 | 21.2 ± 0.3 | 9.1 |

| Bread with brewer's yeast | | |
|---|---|---|
| R | 31.9 ± 0.3 | 5.3 |
| 1 | 32.2 ± 0.4 | 5.2 |
| 4 | 30.9 ± 0.4 | 5.7 |
| 5 | 32.1 ± 0.8 | 5.2 |
| 9 | 38.6 ± 0.5 | 2.4 |
| 10 | 36.4 ± 2.8 | 3.3 |
| 16 | 32.8 ± 1.7 | 4.9 |
| 18 | 32.8 ± 0.6 | 4.9 |
| 19 | 31.9 ± 1.8 | 5.3 |
| 20 | 31.1 ± 1.2 | 5.6 |
| 22 | 31.7 ± 0.9 | 5.4 |
| 23 | 31.5 ± 0.9 | 5.5 |
| 30 | 31.6 ± 0.8 | 5.4 |
| 31 | 34.8 ± 1.3 | 4.0 |
| A10 | 33.4 ± 1.2 | 4.6 |
| A12 | 29.5 ± 0.3 | 6.3 |
| A13 | 32.4 ± 1.0 | 5.1 |
| A14 | 31.1 ± 0.6 | 5.6 |
| A15 | 30.7 ± 0.6 | 5.8 |
| A20 | 31.6 ± 0.6 | 5.4 |
| A22 | 32.1 ± 0.8 | 5.2 |
| A23 | 33.3 ± 2.0 | 4.7 |
| A25 | 31.3 ± 0.3 | 5.5 |
| A27 | 30.9 ± 0.9 | 5.7 |
| A28 | 31.5 ± 0.5 | 5.5 |
| A30 | 30.4 ± 0.7 | 5.9 |
| A33 | 36.1 ± 1.2 | 3.4 |
| A34 | 31.0 ± 1.0 | 5.7 |
| A35 | 31.0 ± 0.8 | 5.7 |
| A48 | 30.6 ± 0.7 | 5.9 |
| A49 | 32.9 ± 0.4 | 4.8 |
| A50 | 33.4 ± 0.3 | 4.6 |
| A45 | 28.5 ± 0.2 | 6.8 |
| A46 | 28.7 ± 0.5 | 6.7 |
| A39 | 31.1 ± 0.8 | 5.6 |
| A38 | 31.5 ± 1.1 | 5.5 |

**Table 7. Ingredients, technological parameters and cellular density of lactic acid bacteria of the lab breads produced with dehydrated sourdough only.**

| **Code** | **Flour (g)** | **Water (g)** | **Dehydrated sourdough (g)** | **Lactic acid bacteria (log cfu/g)** | **Leavening time (h)** |
|---|---|---|---|---|---|
| Brand A | | | | | |
| P1 | 106.7 | 88.9 | 4.4 | | |
| P2 | 102.2 | 88.9 | 8.9 | < 4.0 | 6 |
| P3 | 71.1 | 88.9 | 40.0 | | |

| Brand B | | | | | |
|---|---|---|---|---|---|
| P4 | 106.7 | 88.9 | 4.4 | | |
| P5 | 102.2 | 88.9 | 8.9 | < 4.0 | 6 |
| P6 | 71.1 | 88.9 | 40.0 | | |

| Brand C | | | | | |
|---|---|---|---|---|---|
| P7 | 106.7 | 88.9 | 4.4 | | |
| P8 | 102.2 | 88.9 | 8.9 | < 4.0 | 6 |
| P9 | 71.1 | 88.9 | 40.0 | | |

| Brand D | | | | | |
|---|---|---|---|---|---|
| P10 | 106.7 | 88.9 | 4.4 | | |
| P11 | 102.2 | 88.9 | 8.9 | < 4.0 | 6 |
| P12 | 71.1 | 88.9 | 40.0 | | |

| Brand E | | | | | |
|---|---|---|---|---|---|
| P13 | 106.7 | 88.9 | 4.4 | | |
| P14 | 102.2 | 88.9 | 8.9 | < 4.0 | 6 |
| P15 | 71.1 | 88.9 | 40.0 | | |

| Brand F | | | | | |
|---|---|---|---|---|---|
| P16 | 106.7 | 88.9 | 4.4 | < 4.0 | 6 |
| P17 | 102.2 | 88.9 | 8.9 | | |
| P18 | 71.1 | 88.9 | 40.0 | | |

| Brand G | | | | | |
|---|---|---|---|---|---|
| P19 | 106.7 | 88.9 | 4.4 | | |
| P20 | 102.2 | 88.9 | 8.9 | < 4.0 | 6 |
| P21 | 71.1 | 88.9 | 40.0 | | |

**Table 8. Cycle threshold (C_{T}) and number of copies of the 16S rRNA gene of the lab breads produced with dehydrated sourdough only.**

| **Code** | **Cycle threshold (C_{T})** | **Number of copies of the gene (log gene copies/g)** |
|---|---|---|
| P1 | 30.0 ± 0.1 | 6.1 |
| P2 | 26.8± 0.2 | 7.5 |
| P3 | 26.7± 0.1 | 7.6 |
| P4 | 26.1± 0.2 | 7.8 |
| P5 | 23.6± 0.1 | 8.9 |
| P6 | 21.2± 0.2 | 10.0 |
| P7 | 27.6± 0.1 | 7.2 |
| P8 | 24.7± 0.1 | 8.4 |
| P9 | 21.9± 0.3 | 9.6 |
| P10 | 31.4± 0.1 | 5.5 |
| P11 | 27.8± 0.1 | 7.1 |
| P12 | 22.7± 0.2 | 9.3 |
| P13 | 30.9± 0.3 | 5.7 |
| P14 | 27.2± 0.1 | 7.3 |
| P15 | 26.7± 0.1 | 7.5 |
| P16 | 28.3± 0.1 | 6.8 |
| P17 | 25.1± 0.3 | 8.3 |
| P18 | 23.3± 0.2 | 9.1 |
| P19 | 25.73± 0.1 | 8.0 |
| P20 | 23.25± 0.1 | 9.1 |

| | | |
|---|---|---|
| P21 | 21.53± 0.5 | 9.8 |

### Example 4: Biochemical characterization of the breads employed to optimize and define the efficiency of the quantitative PCR protocol.

A total of 170 breads produced in the lab or purchased at bakeries or supermarkets have been characterized based on the pH values, Total Titratable Acidity (TTA) and lactic and acetic acid concentrations (Table 9). The pH values have been measured by pH-meter (Model 507, Crison, Milan, Italy) equipped with a probe for solid food. The TTA has been measured on 10 g bread homogenized with 90 ml distilled water for 3 minutes in a Bag Mixer 400P (Interscience, St Nom, France). The TTA has been expressed as volume (ml) of 0.1 N NaOH needed to reach a pH value equal to 8.3. The lactic and acetic acids have been determined in the aqueous extract of the breads by using the HPLC ÄKTA Purifier™ system (GE Healthcare Bio-Sciences, Uppsala, Sweden), with detector of the refractive index (Perkin Elmer Corp., Waltham, MA). Table 9. pH values, TTA (ml of 0.1 N NaOH), and lactic and acetic acid concentrations (mmol/kg) of the lab or commercial breads produced with sourdough or brewer's yeast.

| **Code** | **pH** | **TTA (ml 0.1N NaOH)** | **Lactic acid (mmol/kg)** | **Acetic acid (mmol/kg)** |
|---|---|---|---|---|
| Lab bread with sourdough | | | | |
| Vd1 | 6.10 ± 0.01 | 2.4 ± 0.2 | 1.8 ± 0.4 | 1.1 ± 0.3 |
| Vd2 | 6.07 ± 0.03 | 2.6 ± 0.2 | 2.6 ± 0.3 | 1.0 ± 0.2 |
| Vd3 | 6.04 ± 0.02 | 3.0 ± 0.1 | 2.9 ± 0.2 | 1.1 ± 0.2 |
| Vd4 | 5.86 ± 0.03 | 3.0 ± 0.1 | 4.8 ± 0.3 | 4.0 ± 0.4 |
| Vd5 | 5.89 ± 0.02 | 3.2 ± 0.3 | 4.7 ± 0.2 | 2.9 ± 0.3 |
| V1 | 5.76 ± 0.05 | 3.2 ± 0.2 | 7.4 ± 0.3 | 2.5 ± 0.4 |
| V2 | 5.11 ± 0.01 | 4.8 ± 0.3 | 7.5 ± 0.4 | 3.3 ± 0.5 |
| V3 | 4.49 ± 0.03 | 7.2 ± 0.1 | 10.1 ± 0.1 | 11.7 ± 0.6 |
| V4 | 4.27 ± 0.01 | 8.8 ± 0.3 | 12.4 ± 0.1 | 9.8 ± 0.5 |
| V5 | 4.05 ± 0.01 | 11.8 ± 0.2 | 23.4 ± 0.4 | 9.8 ± 0.6 |
| ALAd1 | 6.08 ± 0.04 | 2.2 ± 0.1 | nd | nd |
| ALAd2 | 6.01 ± 0.01 | 2.7 ± 0.2 | 2.9 ± 0.3 | 1.1 ± 0.1 |
| ALAd3 | 6.02 ± 0.02 | 2.9 ± 0.1 | 3.0 ± 0.4 | 1.4 ± 0.1 |
| ALAd4 | 5.88 ± 0.01 | 3.4 ± 0.3 | 3.9 ± 0.2 | 2.4 ± 0.2 |
| ALAd5 | 5.65 ± 0.02 | 3.4 ± 0.1 | 5.9 ± 0.1 | 2.5 ± 0.3 |
| ALA1 | 5.64 ± 0.03 | 3.6 ± 0.2 | 8.4 ± 0.2 | 2.3 ± 0.3 |
| ALA2 | 4.91 ± 0.05 | 6.0 ± 0.1 | 11.5 ± 0.1 | 3.2 ± 0.4 |
| ALA3 | 4.32 ± 0.02 | 8.4 ± 0.2 | 12.9 ± 0.3 | 5.4 ± 0.2 |
| ALA4 | 4.17 ± 0.03 | 10.6 ± 0.1 | 18.6 ± 0.4 | 7.1 ± 0.3 |
| ALA5 | 3.93 ± 0.01 | 13.4 ± 0.2 | 28.8 ± 0.1 | 6.1 ± 0.1 |
| BAd1 | 6.02 ± 0.05 | 3.1 ± 0.3 | 3.2 ± 0.3 | 1.4 ± 0.3 |
| BAd2 | 6.01 ± 0.02 | 3.4 ± 0.3 | 2.8 ± 0.4 | 1.9 ± 0.4 |
| BAd3 | 6.05 ± 0.03 | 3.3 ± 0.2 | 3.1 ± 0.3 | 1.7 ± 0.2 |
| BAd4 | 5.69 ± 0.01 | 3.6 ± 0.1 | 6.0 ± 0.3 | 3.2 ± 0.1 |
| BAd5 | 5.35 ± 0.03 | 3.5 ± 0.1 | 5.2 ± 0.2 | 3.5 ± 0.3 |
| BA1 | 5.47 ± 0.02 | 3.3 ± 0.3 | 5.0 ± 0.1 | 4.0 ± 0.2 |
| BA2 | 4.87 ± 0.03 | 5.4 ± 0.1 | 9.3 ± 0.3 | 9.1 ± 0.3 |
| BA3 | 4.34 ± 0.01 | 8.6 ± 0.2 | 16.2 ± 0.2 | 8.4 ± 0.2 |
| BA4 | 4.18 ± 0.01 | 10.1 ± 0.1 | 19.1 ± 0.4 | 5.9 ± 0.4 |
| BA5 | 4.08 ± 0.02 | 11.1 ± 0.2 | 22.5 ± 0.3 | 11.0 ± 0.3 |
| CG1 | 4.94 ± 0.01 | 6.2 ± 0.2 | 9.0 ± 0.2 | 3.0 ± 0.3 |
| CG2 | 4.65 ± 0.03 | 7.5 ± 0.1 | 9.1 ± 0.2 | 6.4 ± 0.2 |
| CG3 | 4.46 ± 0.04 | 7.4 ± 0.2 | 10.4 ± 0.3 | 9.3 ± 0.1 |
| CG4 | 4.39 ± 0.04 | 8.4 ± 0.2 | 12.3 ± 0.1 | 10.0 ± 0.3 |
| CG5 | 4.38 ± 0.02 | 8.7 ± 0.2 | 12.7 ± 0.3 | 9.3 ± 0.2 |
| CG6 | 4.28 ± 0.02 | 9.1 ± 0.1 | 14.9 ± 0.4 | 8.1 ± 0.3 |
| CG7 | 4.04 ± 0.01 | 11.7 ± 0.3 | 19.8 ± 0.2 | 10.8 ± 0.2 |
| MTA1 | 4.92 ± 0.03 | 6.8 ± 0.1 | 11.9 ± 0.4 | 2.9 ± 0.2 |
| MTA2 | 4.45 ± 0.01 | 8.4 ± 0.2 | 14.8 ± 0.6 | 4.4 ± 0.2 |
| MTA3 | 4.31 ± 0.01 | 9.0 ± 0.2 | 18.3 ± 0.4 | 6.4 ± 0.3 |
| MTA4 | 4.17 ± 0.01 | 10.4 ± 0.1 | 22.0 ± 0.2 | 4.5 ± 0.3 |
| MTA5 | 4.14 ± 0.03 | 10.1 ± 0.1 | 19.8 ± 0.2 | 3.4 ± 0.2 |
| MTA6 | 4.10 ± 0.01 | 11.7 ± 0.3 | 22.7 ± 0.4 | 5.0 ± 0.4 |
| MTA7 | 4.02 ± 0.01 | 12.9 ± 0.1 | 28.8 ± 0.2 | 5.4 ± 0.5 |
| TD1 | 5.22 ± 0.05 | 3.6 ± 0.2 | 6.5 ± 0.2 | 3.1 ± 0.4 |
| TD2 | 4.44 ± 0.02 | 7.4 ± 0.1 | 11.8 ± 0.3 | 5.7 ± 0.4 |
| TD3 | 4.38 ± 0.01 | 8.2 ± 0.1 | 13.1 ± 0.6 | 8.6 ± 0.3 |
| TD4 | 4.34 ± 0.03 | 8.2 ± 0.2 | 13.9 ± 0.3 | 9.6 ± 0.5 |
| TD5 | 4.31 ± 0.03 | 8.4 ± 0.2 | 14.5 ± 0.4 | 5.7 ± 0.4 |
| TD6 | 4.28 ± 0.01 | 8.8 ± 0.3 | 15.9 ± 0.4 | 6.3 ± 0.4 |
| TD7 | 4.24 ± 0.01 | 9.0 ± 0.4 | 16.7 ± 0.3 | 7.3 ± 0.3 |
| TA1 | 4.91 ± 0.02 | 6.1 ± 0.1 | 7.6 ± 0.4 | 4.5 ± 0.4 |
| TA2 | 4.42 ± 0.02 | 7.8 ± 0.1 | 11.8 ± 0.5 | 5.0 ± 0.5 |
| TA3 | 4.31 ± 0.01 | 8.9 ± 0.4 | 14.9 ± 0.4 | 5.5 ± 0.3 |
| TA4 | 4.32 ± 0.03 | 9.0 ± 0.3 | 15.1 ± 0.4 | 7.3 ± 0.4 |
| TA5 | 4.29 ± 0.04 | 9.5 ± 0.2 | 16.7 ± 0.2 | 5.4 ± 0.2 |
| TA6 | 4.17 ± 0.02 | 10.5 ± 0.2 | 17.1 ± 0.4 | 6.6 ± 0.2 |
| TA7 | 4.08 ± 0.04 | 11.2 ± 0.1 | 18.9 ± 0.5 | 2.5 ± 0.2 |
| ALB1 | 4.94 ± 0.02 | 6.4 ± 0.3 | 10.3 ± 0.4 | 6.8 ± 0.4 |
| ALB2 | 4.61 ± 0.04 | 7.4 ± 0.1 | 9.3 ± 0.3 | 8.8 ± 0.5 |
| ALB3 | 4.32 ± 0.04 | 8.5 ± 0.2 | 13.2 ± 0.3 | 9.7 ± 0.5 |
| ALB4 | 4.21 ± 0.05 | 10.4 ± 0.3 | 16.5 ± 0.2 | 8.1 ± 0.2 |
| ALB5 | 4.34 ± 0.02 | 9.0 ± 0.1 | 17.4 ± 0.3 | 6.3 ± 0.6 |
| ALB6 | 4.17 ± 0.02 | 12.2 ± 0.2 | 19.8 ± 0.3 | 7.5 ± 0.6 |
| ALB7 | 4.09 ± 0.01 | 10.8 ± 0.1 | 20.4 ± 0.1 | 9.6 ± 0.5 |
| Lab bread with brewer's yeast | | | | |
| BY1 | 6.11 ± 0.01 | 1.5 ± 0.2 | 0.4 ± 0.2 | 0.5 ± 0.2 |
| BY2 | 6.15 ± 0.05 | 1.4 ± 0.1 | nd | nd |
| BY3 | 6.17 ± 0.01 | 1.1 ± 0.3 | nd | nd |
| BY4 | 5.99 ± 0.02 | 1.7 ± 0.1 | 0.9 ± 0.2 | 0.3 ± 0.1 |
| BY5 | 6.13 ± 0.05 | 1.6 ± 0.2 | nd | nd |
| BY6 | 6.15 ± 0.02 | 1.6 ± 0.2 | nd | nd |
| BY7 | 6.25 ± 0.01 | 0.8 ± 0.1 | nd | nd |
| BY8 | 6.23 ± 0.04 | 0.6 ± 0.1 | nd | nd |
| BY9 | 6.25 ± 0.02 | 0.9 ± 0.1 | nd | nd |
| BY10 | 6.14 ± 0.03 | 1.1 ± 0.1 | 0.1 ± 0.1 | 0.5 ± 0.1 |
| BY11 | 6.17 ± 0.01 | 1.3 ± 0.2 | nd | nd |
| BY12 | 6.21 ± 0.02 | 1.2 ± 0.1 | nd | nd |

| Commercial bread with sourdough | | | | |
|---|---|---|---|---|
| A | 5.01 ± 0.01 | 6.2 ± 0.2 | 9.0 ± 0.2 | 2.2 ± 0.2 |
| B | 4.86 ± 0.01 | 7.0 ± 0.1 | 12.3 ± 0.3 | 9.5 ± 0.4 |
| C | 5.12 ± 0.03 | 5.6 ± 0.2 | 7.3 ± 0.3 | 6.4 ± 0.6 |
| D | 5.11 ± 0.04 | 6.6 ± 0.3 | 8.1 ± 0.5 | 1.0 ± 0.1 |
| E | 5.32 ± 0.02 | 3.5 ± 0.2 | 6.6 ± 0.5 | 3.5 ± 0.3 |
| I | 5.21 ± 0.05 | 6.1 ± 0.2 | 10.6 ± 0.3 | 4.2 ± 0.3 |
| L | 5.02 ± 0.02 | 6.5 ± 0.1 | 9.4 ± 0.4 | 5.0 ± 0.6 |
| M | 5.24 ± 0.02 | 4.4 ± 0.1 | 9.1 ± 0.5 | 7.9 ± 0.4 |
| N | 4.76 ± 0.04 | 7.0 ± 0.3 | 10.2 ± 0.4 | 7.2 ± 0.4 |
| O | 4.96 ± 0.02 | 7.4 ± 0.1 | 16.2 ± 0.6 | 10.6 ± 0.1 |
| P | 5.01 ± 0.01 | 7.8 ± 0.2 | 12.1 ± 0.6 | 9.2 ± 0.5 |
| Q | 5.71 ± 0.02 | 3.0 ± 0.1 | 3.1 ± 0.2 | 0.9 ± 0.1 |
| T | 4.39 ± 0.03 | 7.2 ± 0.3 | 14.2 ± 0.4 | 7.0 ± 0.5 |
| U | 5.01 ± 0.03 | 6.5 ± 0.2 | 9.7 ± 0.2 | 5.1 ± 0.3 |
| V | 4.87 ± 0.05 | 6.6 ± 0.2 | 12.3 ± 0.3 | 9.7 ± 0.3 |
| Z | 5.04 ± 0.01 | 6.7 ± 0.1 | 9.8 ± 0.4 | 6.3 ± 0.4 |
| A2 | 5.01 ± 0.02 | 6.6 ± 0.3 | 10.1 ± 0.2 | 6.2 ± 0.4 |
| A3 | 5.22 ± 0.04 | 5.6 ± 0.2 | 8.1 ± 0.6 | 4.7 ± 0.5 |
| A4 | 4.61 ± 0.02 | 7.8 ± 0.2 | 10.1 ± 0.2 | 7.6 ± 0.3 |
| A5 | 4.91 ± 0.02 | 6.9 ± 0.2 | 11.4 ± 0.2 | 9.4 ± 0.3 |
| A6 | 4.86 ± 0.05 | 7.0 ± 0.1 | 8.2 ± 0.5 | 5.9 ± 0.3 |
| A7 | 4.90 ± 0.01 | 7.1 ± 0.1 | 10.8 ± 0.3 | 11.5 ± 0.3 |
| A8 | 4.95 ± 0.04 | 8.0 ± 0.2 | 10.4 ± 0.4 | 6.3 ± 0.2 |
| A9 | 5.41 ± 0.02 | 4.1 ± 0.1 | 7.2 ± 0.4 | 3.4 ± 0.2 |
| A11 | 5.11 ± 0.02 | 5.7 ± 0.2 | 9.2 ± 0.5 | 2.3 ± 0.3 |
| A16 | 5.65 ± 0.02 | 4.0 ± 0.1 | 7.5 ± 0.3 | 3.8 ± 0.4 |
| A17 | 5.11 ± 0.01 | 5.5 ± 0.1 | 8.7 ± 0.5 | 4.8 ± 0.4 |
| A18 | 5.01 ± 0.06 | 5.0 ± 0.3 | 5.1 ± 0.4 | 3.3 ± 0.1 |
| A19 | 5.49 ± 0.05 | 4.3 ± 0.3 | 10.8 ± 0.3 | 4.8 ± 0.3 |
| A21 | 5.47 ± 0.02 | 4.6 ± 0.4 | 7.7 ± 0.1 | 2.8 ± 0.2 |
| A24 | 5.57 ± 0.05 | 3.2 ± 0.1 | 5.1 ± 0.2 | 2.3 ± 0.2 |
| A26 | 5.47 ± 0.05 | 5.0 ± 0.2 | 9.1 ± 0.3 | 4.2 ± 0.4 |
| A29 | 5.24 ± 0.04 | 4.9 ± 0.1 | 7.2 ± 0.5 | 1.4 ± 0.2 |
| A31 | 5.01 ± 0.01 | 6.5 ± 0.1 | 10.9 ± 0.2 | 4.0 ± 0.1 |
| A32 | 5.34 ± 0.06 | 5.1 ± 0.5 | 7.9 ± 0.4 | 2.3 ± 0.3 |
| A37 | 5.15 ± 0.02 | 5.0 ± 0.1 | 6.2 ± 0.3 | 3.5 ± 0.4 |
| A40 | 4.20 ± 0.04 | 9.0 ± 0.2 | 18.0 ± 0.4 | 7.5 ± 0.3 |
| A41 | 5.15 ± 0.03 | 5.6 ± 0.1 | 9.2 ± 0.3 | 3.6 ± 0.2 |
| A42 | 3.94 ± 0.01 | 12.7 ± 0.4 | 30.4 ± 0.3 | 9.0 ± 0.3 |
| A43 | 5.75 ± 0.01 | 3.0 ± 0.1 | 7.2 ± 0.1 | 0.5 ± 0.2 |
| A44 | 5.18 ± 0.04 | 5.0 ± 0.2 | 9.6 ± 0.2 | 4.8 ± 0.2 |
| A47 | 4.50 ± 0.02 | 7.8 ± 0.3 | 11.2 ± 0.4 | 6.8 ± 0.4 |
| A51 | 5.30 ± 0.01 | 4.0 ± 0.3 | 5.7 ± 0.4 | 3.9 ± 0.2 |
| A52 | 5.20 ± 0.01 | 4.2 ± 0.1 | 5.1 ± 0.3 | 3.2 ± 0.3 |
| 2 | 4.80 ± 0.02 | 5.4 ± 0.3 | 9.0 ± 0.2 | 9.2 ± 0.2 |
| 6 | 5.10 ± 0.04 | 5.4 ± 0.1 | 7.6 ± 0.2 | 4.0 ± 0.1 |
| 8 | 4.94 ± 0.03 | 6.7 ± 0.2 | 10.3 ± 0.3 | 6.0 ± 0.3 |
| 11 | 5.75 ± 0.01 | 2.9 ± 0.1 | 5.5 ± 0.1 | 1.2 ± 0.3 |
| 12 | 5.12 ± 0.02 | 5.1 ± 0.2 | 7.3 ± 0.2 | 5.5 ± 0.4 |
| 13 | 5.14 ± 0.04 | 5.3 ± 0.3 | 8.0 ± 0.1 | 5.2 ± 0.3 |
| 14 | 4.89 ± 0.04 | 5.4 ± 0.2 | 9.9 ± 0.3 | 5.7 ± 0.4 |
| 15 | 5.21 ± 0.02 | 4.4 ± 0.2 | 6.7 ± 0.1 | 3.9 ± 0.4 |
| 26 | 5.26 ± 0.05 | 4.2 ± 0.3 | 6.4 ± 0.3 | 4.2 ± 0.4 |
| 27 | 4.99 ± 0.04 | 6.8 ± 0.1 | 8.3 ± 0.4 | 1.6 ± 0.4 |
| 28 | 5.04 ± 0.02 | 6.1 ± 0.2 | 9.5 ± 0.2 | 1.3 ± 0.3 |
| 29 | 5.01 ± 0.01 | 6.2 ± 0.3 | 7.9 ± 0.3 | 2.1 ± 0.1 |
| 32 | 4.89 ± 0.01 | 6.0 ± 0.3 | 10.3 ± 0.2 | 8.9 ± 0.3 |
| Commercial breads with brewer's yeast | | | | |
| R | 6.00 ± 0.05 | 3.0 ± 0.1 | nd | nd |
| 1 | 6.30 ± 0.03 | 2.0 ± 0.2 | 1.1 ± 0.1 | 1.6 ± 0.3 |
| 4 | 6.30 ± 0.02 | 1.6 ± 0.3 | 1.7 ± 0.2 | 1.6 ± 0.3 |
| 5 | 6.20 ± 0.04 | 2.0 ± 0.2 | nd | nd |
| 9 | 6.20 ± 0.04 | 2.0 ± 0.2 | 1.2 ± 0.4 | 0.5 ± 0.4 |
| 10 | 6.00 ± 0.04 | 2.4 ± 0.1 | 1.3 ± 0.2 | 1.2 ± 0.4 |
| 16 | 5.90 ± 0.01 | 3.1 ± 0.2 | 1.2 ± 0.4 | 1.5 ± 0.3 |
| 18 | 6.20 ± 0.02 | 2.2 ± 0.3 | nd | nd |
| 19 | 6.40 ± 0.02 | 2.8 ± 0.1 | nd | nd |
| 20 | 6.00 ± 0.02 | 2.0 ± 0.3 | nd | nd |
| 22 | 6.20 ± 0.03 | 1.8 ± 0.2 | nd | nd |
| 23 | 6.30 ± 0.02 | 1.4 ± 0.3 | nd | nd |
| 30 | 6.10 ± 0.03 | 2.0 ± 0.1 | 1.1 ± 0.2 | 1.5 ± 0.4 |
| 31 | 6.00 ± 0.02 | 1.6 ± 0.3 | nd | nd |
| A10 | 6.05 ± 0.01 | 2.5 ± 0.1 | nd | nd |
| A12 | 5.85 ± 0.01 | 3.1 ± 0.2 | nd | nd |
| A13 | 5.89 ± 0.01 | 3.0 ± 0.2 | nd | nd |
| A14 | 5.85 ± 0.04 | 3.0 ± 0.1 | nd | nd |
| A15 | 5.99 ± 0.04 | 3.0 ± 0.3 | nd | nd |
| A20 | 6.01 ± 0.04 | 3.0 ± 0.1 | nd | nd |
| A22 | 6.03 ± 0.05 | 2.0 ± 0.1 | nd | nd |
| A23 | 5.97 ± 0.06 | 3.1 ± 0.3 | nd | nd |
| A25 | 5.92 ± 0.02 | 3.0 ± 0.2 | nd | nd |
| A27 | 6.12 ± 0.02 | 3.0 ± 0.2 | nd | nd |
| A28 | 6.00 ± 0.04 | 2.4 ± 0.1 | nd | nd |
| A30 | 6.12 ± 0.02 | 3.8 ± 0.2 | nd | nd |
| A33 | 5.93 ± 0.04 | 3.0 ± 0.3 | nd | nd |
| A34 | 5.95 ± 0.04 | 2.8 ± 0.1 | nd | nd |
| A35 | 6.05 ± 0.03 | 1.6 ± 0.1 | nd | nd |
| A48 | 6.10 ± 0.03 | 1.2 ± 0.2 | nd | nd |
| A49 | 5.87 ± 0.05 | 3.2 ± 0.1 | nd | nd |
| A50 | 6.10 ± 0.02 | 1.0 ± 0.2 | nd | nd |
| A45 | 5.96 ± 0.02 | 2.2 ± 0.1 | nd | nd |
| A46 | 5.95 ±0.04 | 2.6 ± 0.3 | nd | nd |
| A39 | 5.90 ± 0.02 | 1.4 ± 0.2 | nd | nd |
| A38 | 5.94 ± 0.02 | 2.0 ± 0.3 | nd | nd |

| | | | | |
|---|---|---|---|---|
| N.D.: Not Detectable | | | | |

### Example 5 Statistical processing of the number of copies of gene and biochemical characteristics of the lab and commercial breads.

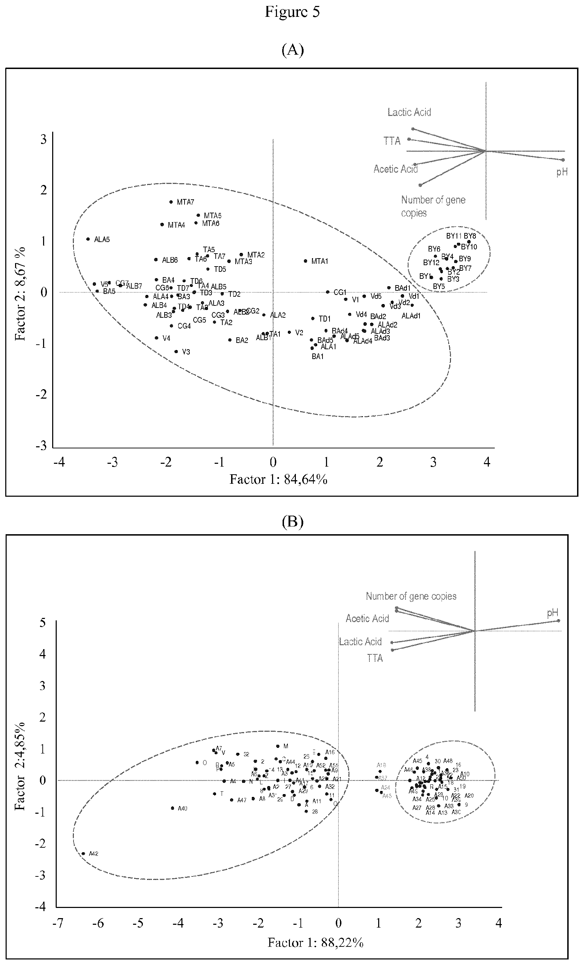

The values of number of copies of the 16S rRNA gene, pH, TTA and the lactic and acetic acid concentrations of the lab (Figure 5A) and commercial breads (Figure 5B) have been subjected to Principal Component Analysis (PCA). The two principal components accounted for 84.64 % (PC1) and 8.67% (PC2) of the total variance of the data. The lab breads produced with brewer's yeast have been grouped in the same area of the plane (red circle), because they showed the lowest values of number of copies of the 16S rRNA gene (< 7 log gene copies/g) and pH (5.99 - 6.25), and the lowest values of TTA (0.6 - 1.7 ml of 0.1N NaOH) and organic acid concentration (< 1.0 mmol/kg). By increasing the value of the number of copies of the 16S rRNA gene, the breads with natural leavening have been clearly separated in another area of the plane (blue circle). The dispersion of these samples was mainly due to the variation of the pH values (3.93 - 5.69), TTA (3.0 - 13.4 ml 0.1 N NaOH) and concentration of lactic and acetic acid (4.7 - 28.8 and 2.3 - 11.7 mmol/kg, respectively). The breads with natural leavening with the highest values of number of copies of the 16S rRNA gene and the highest acidity are distributed at the left end of the plane. Even more evident is the differentiation for the commercial breads (Figure 5B). The two principal components accounted for 93.1% of the total variance of the data. The breads produced with brewer's yeast (red oval) have been separated in the right area of the graph (values of the number of copies of the 16S rRNA gene < 7 log gene copies/g, pH 5.85 - 6.40, TTA 1.0 - 3.2 ml 0.1 N NaOH and concentration of organic acids < 2 mmol/kg). By increasing the value of the number of copies of the 16S rRNA gene above 7 log gene copies/g, the breads with natural leavening (blue oval) were grouped in the left part of the plane. Within this group, the breads coded Q and A42 represented the extremes, because they are respectively characterized by the highest and lowest pH values. The breads without packaging, and thus free of any label, coded A18, A24, A37 and A43 (all depicted in green), even if sold with natural leavening, were grouped separately.

Finally, the obtained set of data has been analyzed by the Pearson correlation index. As expected, the correlations between pH and TTA (-0.954) and lactic acid (-0.922) and acetic acid (-0.840) concentrations was very high for all the samples. On the other hand, the correlation between pH and C_{T} has been rather weak (0.740). This result on one hand confirms the limited effectiveness of the indirect parameters (pH, TTA and organic acids) used to date in the authentication of breads with natural leavening and, on the other hand, emphasizes the need to have molecular methods able to determine, directly on the bread, the cellular density of lactic acid bacteria reached at the end of the leavening process.

Figure 5 depicts the Principal Component Analysis (PCA) based on the values of number of copies of the 16S rRNA gene (log gene copies/g), pH, TTA (ml 0.1 N NaOH) and lactic and acetic acid concentrations (mmol/kg) of lab (A) and commercial (B) breads with natural leavening or produced by using brewer's yeast only.

## Claims

1. A method for the detection of lactic acid bacteria in leavened food products, comprising:
a. extracting the total DNA from a sample of said leavened food product;
b. carrying out a quantitative polymerase chain reaction (qPCR) on the DNA extracted in step (a) by using primers specific for bacterial 16S rRNA sequences, said primer pair being SKfw (5'-GGGGATAACAYYTGGAAACAG-3') (SEQ. ID NO. 1) and SKrw (5'-CTCGGCTACGTATCATTGTCTTG-3') (SEQ. ID NO. 2).
c. determining the CT value;
d. determining, by means of the equation of the calibration line, the value of the number of copies of the 16S rRNA gene expressed as log gene copies/g, wherein the value of the number of copies of the 16S rRNA > 7 log gene copies/g identifies a food product leavened with sourdough.

2. The method according to claim 1, **characterized in that** the determination of the value is carried out by setting the threshold to a value between 0.1 and 1.

3. The method according to any one of claims 1 or 2, **characterized in that** said food product is bread.

4. The method according to any one of claims 1 to 3, **characterized in that** the amplification process comprises:
- -a reaction mixture (25 µl) constituted by: (i) 12.5 µl of Rotor-Gene SYBR Green RT-PCR master mix, (ii) 0.75 µl of each primer (SKfw/SKrw) at a concentration of 100 µM, (iii) 10 µl of RNase free water and (iv) 1 µl of DNA;
- an amplification cycle carried out in the Rotor-Gene 6000 (Corbett Research Ltd,, Australia) which includes a first denaturation step of the DNA at 95°C for 10 minutes followed by 35 cycles of: (i) denaturation at 95°C for 10 seconds; (ii) annealing at 55°C for 30 seconds; and (iii) elongation at 72°C for 30 seconds and, to complete the amplification, a "melting curve" in a temperature range of 60 - 95°C, with 1°C increments;
- a quantification carried out by using the Rotor-Gene 6000 Series Software, version 1.7, by manually setting the "threshold" at a value of 0.4.

5. Oligonucleotides of sequences:
SKfw (5'-GGGGATAACAYYTGGAAACAG-3') (SEQ. ID NO. 1) and
SKrw (5'-CTCGGCTACGTATCATTGTCTTG-3') (SEQ. ID NO. 2)

6. Use of the oligonucleotides according to claim 5 as PCR primers for the detection of bacteria.

7. Kit for the detection and quantitative determination of bacteria in leavened food products, comprising the primer pair
SKfw (5'-GGGGATAACAYYTGGAAACAG-3') (SEQ. ID NO. 1) and
SKrw (5'-CTCGGCTACGTATCATTGTCTTG-3') (SEQ. ID NO. 2).

## Patentansprüche

1. Ein Verfahren zum Nachweis von Milchsäurebakterien in Sauerteig-Lebensmittelprodukten, umfassend:
a. Extrahieren der gesamten DNA aus einer Probe des Sauerteig-Lebensmittelprodukts;
b. Durchführen einer quantitativen Polymerasekettenreaktion (qPCR) an der in Schritt (a) extrahierten DNA unter Verwendung von Primern, die für bakterielle 16S-rRNA-Sequenzen spezifisch sind, wobei die Primer SKfw (5'-GGGGATAACAYYTGGAAACAG-3') (SEQ. ID NO. 1) und SKrw (5'-CTCGGCTACGTATCATTGTCTTG-3') (SEQ. ID NO. 2) sind.
c. Bestimmen des CT-Wertes;
d. Bestimmen mittels der Gleichung der Kalibrierungslinie den Wert der Anzahl von Kopien des 16S-rRNA-Gens, ausgedrückt als log Genkopien/g, wobei der Wert der Anzahl von Kopien der 16S-rRNA > 7 log Genkopien/g ein mit Sauerteig gesäuertes Lebensmittelprodukt identifiziert.

2. Das Verfahren gemäß Anspruch 1, **dadurch gekennzeichnet, dass** die Bestimmung des Wertes durch Einstellen des Schwellenwerts auf einen Wert zwischen 0,1 und 1 erfolgt.

3. Das Verfahren gemäß irgendeinem der Ansprüche 1 oder 2, **dadurch gekennzeichnet, dass** das Lebensmittelprodukt Brot ist.

4. Das Verfahren gemäß irgendeinem der Ansprüche 1 bis 3, **dadurch gekennzeichnet, dass** der Amplifikationsprozess umfasst:
- ein Reaktionsgemisch (25 µl) bestehend aus: (i) 12,5 µl Rotor-Gene SYBR Green RT-PCR-Mastermix, (ii) 0,75 µl jedes Primers (SKfw/SKrw) in einer Konzentration von 100 µM, (iii) 10 µl RNase-freies Wasser und (iv) 1 µl DNA;
- ein Amplifikationszyklus, der im Rotor-Gene 6000 (Corbett Research Ltd, Australien) durchgeführt wird und einen ersten Denaturierungsschritt der DNA bei 95 °C für 10 Minuten umfasst, gefolgt von 35 Zyklen von: (i) Denaturierung bei 95 °C für 10 Sekunden; (ii) Annealing bei 55°C für 30 Sekunden; und (iii) Kettenverlängerung bei 72 °C für 30 Sekunden und zur Vervollständigung der Amplifikation eine "Schmelzkurve" in einem Temperaturbereich von 60 bis 95 °C mit Schritten von 1 °C;
- eine Quantifizierung unter Verwendung der Rotor-Gene 6000 Series-Software, Version 1.7, durch manuelles Einstellen des "Schwellenwerts" auf einen Wert von 0,4.

5. Oligonukleotide der Sequenzen:
SKfw (5'-GGGGATAACAYYTGGAAACAG-3') (SEQ. ID NO. 1) und
SKrw (5'-CTCGGCTACGTATCATTGTCTTG-3') (SEQ. ID NO. 2).

6. Verwendung der Oligonukleotide gemäß Anspruch 5 als PCR-Primer zum Nachweis von Bakterien.

7. Kit zum Nachweis und zur quantitativen Bestimmung von Bakterien in Sauerteig-Lebensmittelprodukten, umfassend das Primerpaar
SKfw (5'-GGGGATAACAYYTGGAAACAG-3') (SEQ. ID NO. 1) und
SKrw (5'-CTCGGCTACGTATCATTGTCTTG-3') (SEQ. ID NO. 2).

## Revendications

1. Procédé pour la détection de bactéries lactiques dans des produits alimentaires levés, comprenant les étapes consistant à :
a. extraire l'ADN total d'un échantillon dudit produit alimentaire levé ;
b. exécuter une réaction en chaîne par polymérase quantitative (qPCR) sur l'ADN extrait à l'étape (a) en utilisant des amorces spécifiques pour des séquences d'ARNr 16S bactériennes, ladite paire d'amorces étant SKfw (5'-GGGGATAACAYYTGGAAACAG-3') (SEQ ID N° : 1) et SKrw (5'-CTCGGCTACGTATCATTGTCTTG-3') (SEQ ID N° : 2) ;
c. déterminer la valeur CT ;
d. déterminer, au moyen de l'équation de la ligne d'étalonnage, la valeur du nombre de copies du gène d'ARNr 16S exprimé comme copies de gène log/g, dans lequel la valeur du nombre de copies de l'ARNr 16S > 7 copies de gène log/g identifie un produit alimentaire levé avec du levain.

2. Procédé selon la revendication 1, **caractérisé en ce que** la détermination de la valeur est exécutée en définissant le seuil sur une valeur entre 0,1 et 1.

3. Procédé selon l'une quelconque des revendications 1 ou 2, **caractérisé en ce que** ledit produit alimentaire est du pain.

4. Procédé selon l'une quelconque des revendications 1 à 3, **caractérisé en ce que** le processus d'amplification comprend :
- un mélange de réaction (25 µl) constitué par : (i) 12,5 µl de mélange maître pour RT-PCR Rotor-Gene SYBR Green, (ii) 0,75 µl de chaque amorce (SKfw/SKrw) à une concentration de 100 µM, (iii) 10 µl d'eau exempte de RNase et (iv) 1 µl d'ADN ;
- un cycle d'amplification exécuté dans le Rotor-Gene 6000 (Corbett Research Ltd., Australie) qui inclut un première étape de dénaturation de l'ADN à 95 °C pendant 10 minutes suivi de 35 cycles de : (i) dénaturation à 95 °C pendant 10 secondes ; (ii) recuit à 55 °C pendant 30 secondes ; et (iii) élongation à 72 °C pendant 30 secondes et, pour achever l'amplification, une « courbe de fusion » dans une plage de températures de 60 à 95 °C, avec des incréments de 1 °C ;
- une quantification exécutée en utilisant le Rotor-Gene 6000 Series Software, version 1.7, en définissant manuellement le « seuil » à une valeur de 0,4.

5. Oligonucléotides de séquences :
SKfw (5'-GGGGATAACAYYTGGAAACAG-3') (SEQ ID N° : 1) et
SKrw (5'-CTCGGCTACGTATCATTGTCTTG-3') (SEQ ID N° : 2).

6. Utilisation des oligonucléotides selon la revendication 5 en tant qu'amorces PCR pour la détection de bactéries.

7. Kit pour la détection et la détermination quantitative de bactéries dans des produits alimentaires levés, comprenant la paire d'amorces
SKfw (5'-GGGGATAACAYYTGGAAACAG-3') (SEQ ID N° : 1) et
SKrw (5'-CTCGGCTACGTATCATTGTCTTG-3') (SEQ ID N° : 2).
